# EUROPÄISCHE PATENTSCHRIFT

(11) **EP 2 768 543 B1**
(45) Veröffentlichungstag und Bekanntmachung des Hinweises auf die Patenterteilung: **30.05.2018**
(21) Anmeldenummer: 12775678.1
(22) Anmeldetag: 19.10.2012
(51) Int. Cl.: A61L 27/02, A61L 27/10, A61L 27/12

(54) **ZUBEREITUNG ZUR HERSTELLUNG EINES IMPLANTATS**
PREPARATION FOR THE MANUFACTURE OF AN IMPLANT
PRÉPARATION POUR LA FABRICATION D'UN IMPLANT

(30) Priorität: 19.10.2011 DE 102011084801
(43) Veröffentlichungstag der Anmeldung: 27.08.2014
(73) Patentinhaber: InnoTERE GmbH, 01445 Radebeul (DE)
(72) Erfinder: NIES, Berthold, 64407 Fränkisch-Crumbach (DE)
(74) Vertreter: Kailuweit & Uhlemann Patentanwälte Partnerschaft mbB
(86) Internationale Anmeldenummer: PCT/EP2012/070792
(87) Internationale Veröffentlichungsnummer: WO 2013/057271

(56) Entgegenhaltungen:
- DE-A1-102008 028 738
- US-A1- 2008 028 992
- US-A1- 2009 048 145
- US-A1- 2011 152 195
- US-B1- 6 497 901

## Beschreibung

Die Erfindung betrifft eine Zubereitung zur Herstellung eines Implantats, vorzugsweise eines Knochenimplantats, Verfahren zu dessen Herstellung sowie daraus erhältliche Formkörper. Die Erfindung betrifft auch die Verwendung der Zubereitung als Material zur Behandlung von Knochendefekten oder zum Knochenaufbau, zur Befestigung von Knochenimplantaten oder zur Herstellung von implantierbaren Wirkstoffträgern.

Mineralische Zemente, beispielsweise Calciumphosphat- und Magnesiumphosphatzemente, die zur Herstellung von Implantaten eingesetzt werden, werden üblicherweise in Pulverform bereitgestellt. Aufgrund der durch das Vermischen des Pulvers mit Wasser ausgelösten Abbindereaktion wird ein Festkörper gebildet. Um die Handhabung mineralischer Zemente und die Anmischung der mineralischen Zemente mit Wasser zu erleichtern, werden Zubereitungen vorgeschlagen, in denen mineralische Zementpulver in einer Trägerflüssigkeit dispergiert sind. Die dadurch erhältliche Paste ist lagerstabil, ermöglicht eine einfache Dosierung des mineralischen Zementmaterials und ist leicht mit Wasser anzumischen.

JP 01 139516 A offenbart Pasten zur Anwendung als Zahnfüllmaterial, die organische Trägerflüssigkeiten und mineralische Zementpulver enthalten. Durch die Anwesenheit der Trägerflüssigkeit in der Paste entfällt eine separate Anmischung mit Wasser vor der Anwendung. Als Trägerflüssigkeiten werden vorzugsweise Pflanzenöl, Polyalkohole, Polyglykole, Silikonöle und dickflüssige Paraffine eingesetzt. Der Feststoffgehalt der Pasten beträgt etwa 66 %. Die Pasten härten nach mehrstündigem Kontakt mit Wasser aus. Die durch die Aushärtung der Pasten erhältlichen Formkörper weisen eine Druckfestigkeit von maximal etwa 6 MPa auf (1 kg/cm² entspricht 0,1 MPa).
DE 10 2008 028 738 A1 offenbart Pasten zur Herstellung von Knochenimplantaten, bei denen mineralische Knochenzementpulver in einer oder mehreren wasserfreien und nicht wasserlöslichen Trägerflüssigkeiten enthalten sind. Zur Verbesserung der Einmischung der Feststoffe in die Paste und zur Erleichterung der Anmischung der Paste mit Wasser werden bevorzugt Tenside zugesetzt. Trotz des Einsatzes wasserfreier Trägerflüssigkeiten genügt zur Auslösung der Abbindereaktion das Einbringen der Paste in Wasser. Es werden Pasten mit Feststoffgehalten von bis zu 81 % beschrieben, wobei u.a. Tween und Amphisol als Tenside zugesetzt werden. Die Aushärtezeit der Zementzubereitungen beträgt wenige Minuten. Die Druckfestigkeiten der nach Aushärten erhältlichen Formkörper bei Verwendung von Calciumphosphatzementen betrugen maximal 14 MPa. Für Formkörper mit Magnesiumphosphatzementen wurden Druckfestigkeiten von bis zu 23 MPa erzielt.

US 2011/152195 A1 offenbart ein Knochenzementsystem beinhaltend einen ersten Behälter mit einer wässrigen sauren Paste, die zumindest eine saure Calciumphosphatverbindung, mindestens ein polymerbasiertes Stabilisierungsreagenz, einen pH-Puffer und ein Feuchthaltemittel enthält und einen zweiten Behälter, der eine alkalische nicht wässrige Paste aufweisend mindestens eine basische Calciumphosphatverbindung, mindestens ein Stabilisierungsreagenz, ein Tensid und ein Lösemittel enthält.
Es besteht weiter Bedarf an verbesserten Zementzubereitungen, mit denen hochfeste Formkörper nach einer geringen Aushärtezeit erhältlich sind. Insbesondere besteht Bedarf, Zementzubereitungen mit resorbierbaren mineralischen Knochenzementen, insbesondere Calciumphosphatzementen, mit verbesserter Druckfestigkeit bereitzustellen.
Aufgabe der Erfindung ist es, eine Zubereitung zur Herstellung eines Implantats bereitzustellen, mit denen Calciumionen-haltige und/oder Magnesiumionen-haltige Implantate, insbesondere calciumphosphathaltige Implantate, mit einer hohen Druckfestigkeit erzeugt werden können. Eine weitere Aufgabe der Erfindung besteht darin, eine pastöse, im Wesentlichen wasserfreie und lagerstabile Zubereitung zur Herstellung eines Implantats bereitzustellen, die auch ohne intensive Vermischung mit Wasser zur Aushärtung gebracht werden kann, wobei solide Formkörper mit hoher Druckfestigkeit gebildet werden.
Die Aufgabe wird erfindungsgemäß gelöst durch eine Zubereitung zur Herstellung eines Implantats, vorzugsweise Knochenimplantats, umfassend die folgenden Bestandteile:
a) mineralisches Zementpulver, das mindestens eine Calciumionen-haltige und/oder mindestens eine Magnesiumionen-haltige anorganische Verbindung als Reaktivkomponente enthält,
b) mindestens eine organische Trägerflüssigkeit,
   wobei die organische Trägerflüssigkeit eine schwer wasserlösliche Trägerflüssigkeit ist, wobei die schwer wasserlösliche Trägerflüssigkeit ausgewählt ist aus kurz- oder mittelkettigen Triglyceriden,
   wobei kurzkettige Triglyceride Verbindungen von Fettsäuren einer Länge von jeweils 2 bis 5 Kohlenstoffatomen sind,
   wobei mittelkettige Triglyceride Verbindungen von Fettsäuren einer Länge von jeweils 6 bis 14 Kohlenstoffatomen sind,
c) mindestens zwei Tenside ausgewählt aus mindestens zwei der Gruppen der anionischen, kationischen, amphoteren und nichtionischen Tenside,
d) weniger als 1 Gew.-% Wasser bezogen auf die Gesamtmasse der Zubereitung, dadurch gekennzeichnet,
dass das Gewichtsverhältnis der insgesamt in der Zubereitung enthaltenen Feststoffe zur Summe des Gewichts der organischen Trägerflüssigkeit und der mindestens zwei Tenside mehr als 5 beträgt, wenn das Zementpulver Calciumionen-haltige Verbindungen und außer als Verunreinigungen enthaltene Anteile von Magnesiumverbindungen keine Magnesiumionen-haltigen Verbindungen als Reaktivkomponente enthält, oder mehr als 6 beträgt, wenn das Zementpulver Magnesiumionen-haltige oder Magnesiumionen- und Calciumionen-haltige Verbindungen als Reaktivkomponente enthält,
dass mindestens ein anionisches und mindestens ein nichtionisches Tensid enthalten ist, wobei das mindestens eine anionisches und mindestens eine nichtionische Tensid mindestens ein ethoxylierter Fettalkohol, mindestens eine ethoxylierte Fettsäure, mindestens ein ethoxylierter Sorbitanfettsäureester, mindestens ein ethoxyliertes Fett oder mindestens ein ethoxyliertes Öl und
mindestens ein mit Zitronensäure, Schwefelsäure oder Phosphorsäure veresterter Fettalkohol, mindestens ein mit Zitronensäure, Schwefelsäure oder Phosphorsäure verestertes Mono- oder Diglycerid oder Salz davon oder mindestens eine mit Zitronensäure, Schwefelsäure oder Phosphorsäure veresterte Fettsäure oder Salze davon sind.

Enthält das Zementpulver Calciumionen-haltige Verbindungen und keine Magnesiumionen-haltigen Verbindungen als Reaktivkomponente heißt das, dass dieser Zubereitung keine Magnesiumionen-haltigen Verbindungen als Reaktivkomponente zugesetzt werden. Die Calciumionen-haltigen Verbindungen können aber handelsübliche Verunreinigungen oder Beimengungen Magnesiumionen-haltiger Verbindungen, d. h. max. 1 Gew.-%, bevorzugt max. 0,5 Gew.-% Magnesiumverbindungen aufweisen.

Nach einer vorteilhaften Ausgestaltung beinhaltet die Erfindung eine Calciumionen-haltige Zubereitung zur Herstellung eines Implantats, vorzugsweise Knochenimplantats, die die folgenden Bestandteile umfasst:
a) mineralisches Zementpulver, vorzugsweise mineralisches Knochenzementpulver, mit Calciumionen-haltigen anorganischen Verbindungen als Reaktivkomponente,
b) mindestens eine organische Trägerflüssigkeit,
   wobei die organische Trägerflüssigkeit eine schwer wasserlösliche Trägerflüssigkeit ist,
   wobei die schwer wasserlösliche Trägerflüssigkeit ausgewählt ist aus kurz- oder mittelkettigen Triglyceriden,
   wobei kurzkettige Triglyceride Verbindungen von Fettsäuren einer Länge von jeweils 2 bis 5 Kohlenstoffatomen sind,
c) wobei mittelkettige Triglyceride Verbindungen von Fettsäuren einer Länge von jeweils 6 bis 14 Kohlenstoffatomen sind, mindestens zwei Tenside ausgewählt aus mindestens zwei der Gruppen der anionischen, kationischen, amphoteren und nichtionischen Tenside,
d) weniger als 1 Gew.-%, vorzugsweise weniger als 0,1 Gew.-%, Wasser (bezogen auf die Gesamtmasse der Zubereitung),
   dadurch gekennzeichnet, dass mindestens ein anionisches und mindestens ein nichtionisches Tensid enthalten ist,
   wobei das mindestens eine anionisches und mindestens eine nichtionische Tensid mindestens ein ethoxylierter Fettalkohol, mindestens eine ethoxylierte Fettsäure, mindestens ein ethoxylierter Sorbitanfettsäureester, mindestens ein ethoxyliertes Fett oder mindestens ein ethoxyliertes Öl und
   mindestens ein mit Zitronensäure, Schwefelsäure oder Phosphorsäure veresterter Fettalkohol, mindestens ein mit Zitronensäure, Schwefelsäure oder Phosphorsäure verestertes Mono- oder Diglycerid oder Salz davon oder mindestens eine mit Zitronensäure, Schwefelsäure oder Phosphorsäure veresterte Fettsäure oder Salze davon sind.
In dieser erfindungsgemäßen Zubereitung beträgt das Gewichtsverhältnis der insgesamt in der Zubereitung enthaltenen Feststoffe zur Summe des Gewichts der organischen Trägerflüssigkeit und der mindestens zwei Tenside mehr als 5. Eine derartige Zubereitung enthält keine Magnesiumionen-haltigen Verbindungen als Reaktivkomponente, außer ggf. als Verunreinigungen enthaltene Anteile von Magnesiumverbindungen und wird hierin auch vereinfacht als "Calciumionen-haltige Zubereitung" bezeichnet.

Nach einer vorteilhaften Ausgestaltung beinhaltet die Erfindung eine Magnesiumionen-haltige Zubereitung zur Herstellung eines Implantats, vorzugsweise Knochenimplantats, die die folgenden Bestandteile umfasst:
a) Magnesiumionen-haltiges mineralisches Zementpulver, vorzugsweise Magnesium- und Calciumionen-haltiges mineralisches Zement- bzw. Knochenzementpulver,
b) mindestens eine organische Trägerflüssigkeit,
   wobei die organische Trägerflüssigkeit eine schwer wasserlösliche Trägerflüssigkeit ist,
   wobei die schwer wasserlösliche Trägerflüssigkeit ausgewählt ist aus kurz- oder mittelkettigen Triglyceriden,
   wobei kurzkettige Triglyceride Verbindungen von Fettsäuren einer Länge von jeweils 2 bis 5 Kohlenstoffatomen sind,
   wobei mittelkettige Triglyceride Verbindungen von Fettsäuren einer Länge von jeweils 6 bis 14 Kohlenstoffatomen sind,
c) mindestens zwei Tenside ausgewählt aus mindestens zwei der Gruppen der anionischen, kationischen, amphoteren und nichtionischen Tenside,
d) weniger als 1 Gew.-%, vorzugsweise weniger als 0,1 Gew.-%, Wasser (bezogen auf die Gesamtmasse der Zubereitung),
   dadurch gekennzeichnet, dass mindestens ein anionisches und mindestens ein nichtionisches Tensid enthalten ist,
   wobei das mindestens eine anionisches und mindestens eine nichtionische Tensid mindestens ein ethoxylierter Fettalkohol, mindestens eine ethoxylierte Fettsäure, mindestens ein ethoxylierter Sorbitanfettsäureester, mindestens ein ethoxyliertes Fett oder mindestens ein ethoxyliertes Öl und
   mindestens ein mit Zitronensäure, Schwefelsäure oder Phosphorsäure veresterter Fettalkohol, mindestens ein mit Zitronensäure, Schwefelsäure oder Phosphorsäure verestertes Mono- oder Diglycerid oder Salz davon oder mindestens eine mit Zitronensäure, Schwefelsäure oder Phosphorsäure veresterte Fettsäure oder Salze davon sind.
In einer derartigen erfindungsgemäßen Zubereitung, die mineralisches Zementpulver mit Magnesiumionen-haltigen oder Magnesiumionen- und Calciumionen-haltigen Verbindungen als Reaktivkomponente enthält, beträgt das Gewichtsverhältnis der insgesamt in der Zubereitung enthaltenen Feststoffe zur Summe des Gewichts der organischen Trägerflüssigkeit und der mindestens zwei Tenside mehr als 6. Eine derartige Zubereitung wird hierin auch vereinfacht als "Magnesiumionen-haltige Zubereitung" bezeichnet.

Die erfindungsgemäße Zubereitung liegt in pastöser Form vor. Die Eigenschaften der Paste bleiben bei trockener Lagerung für mindestens 12 Monate stabil. Die Lagerung der Zubereitung hat auf die Eigenschaften des nach Kontakt mit Wasser erhältlichen Formkörpers keinen negativen Einfluss (vergleichbare Eigenschaften des Formkörpers). Durch die Einstellung des Gewichtsverhältnisses zwischen den in der Zubereitung enthaltenen Feststoffen und der Trägerflüssigkeit und der Tenside ist es vorteilhaft möglich, pastöse Zubereitungen bereitzustellen, die nach Aushärtung in einem wässrigen Medium Formkörper mit hohen Druckfestigkeiten von mehr als 25 MPa, von bis zu 40 MPa und in besonderen bevorzugten Formulierungen von bis zu 75 MPa erreichen. Dabei hat sich für Calciumionen-haltige mineralische Zementpulver ein Gewichtsverhältnis von Feststoffen zur Summe des Gewichts der Trägerflüssigkeit und der mindestens zwei Tenside von mehr als 5 und für Magnesiumionen-haltige mineralische Zementpulver ein Gewichtsverhältnis von mehr als 6 als besonders geeignet herausgestellt.

Die erfindungsgemäßen Zubereitungen sind wasserfrei, d.h. es ist weniger als 1 Gew.-% Wasser, bevorzugt weniger als 0,1 Gew.-%, Wasser in der Zubereitung enthalten.

Die Druckfestigkeit der nach Aushärtung der erfindungsgemäßen Zubereitungen in einem wässrigen Medium erhältlichen Formkörper wird anhand von stehenden Formkörpern der Dimension 6x6x12 mm entlang deren längster Achse mit einer Universalprüfmaschine bei einer Vorschubgeschwindigkeit von 1,0 mm/s bestimmt. Die Formkörper werden hergestellt, indem die jeweiligen erfindungsgemäßen Zubereitungen in nach oben offene Formen eingebracht und diese anschließend in eine wässrige 0,9%ige NaCl-Lösung eingelegt werden. Die Messung der Druckfestigkeit der Formkörper erfolgt nach viertägiger Inkubation in der NaCl-Lösung, wobei zunächst die mit den erfindungsgemäßen Zubereitungen gefüllten Formen für 24 h bei 37°C inkubiert und anschließend die entnommenen Formkörper für weitere 72 h bei 37°C inkubiert wurden. Die Messung erfolgt unmittelbar nach der Inkubation (also nach ca. 96 h) im noch feuchten Zustand.
Nach Aushärtung der erfindungsgemäßen Zubereitungen erhaltene Formkörper erreichen eine so bestimmte Druckfestigkeit von 25 bis 75 MPa.

Erfindungsgemäße Zubereitungen enthalten mineralische Zementpulver, vorzugsweise mineralische Knochenzementpulver. Unter mineralischen Zementpulvern werden im Sinne der Erfindung mineralische Feststoffe verstanden, die mit Wasser unter Bildung eines schwerlöslichen Festkörpers reagieren. Bevorzugt sind hydraulisch abbindende mineralische Zementpulver. Bevorzugt enthält das mineralische Zementpulver Silikate, Phosphate, Sulfate, Carbonate, Oxide und/oder Hydroxide, vorzugsweise in Verbindung mit Calcium- und/oder Magnesiumionen als mit Wasser abbindbarer Reaktivkomponente. Bevorzugt enthält das mineralische Zementpulver Calcium- und/oder Magnesiumsalze der Ortho-Phosphorsäure, der dimeren oder polymeren Phosphorsäure, Glycerophosphorsäure und weiterer mono- oder disubstituierter organischer Phosphorsäureester, besonders bevorzugt sind Calcium- und/oder Magnesiumsalze der Ortho-Phosphorsäure. Besonders bevorzugt enthält das mineralische Zementpulver mindestens eine der folgenden Verbindungen: Monocalciumphosphat Monohydrat, Monocalciumphosphat Anhydrit, Dicalciumphosphat Anhydrit, Dicalciumphosphat Dihydrat, Octacalciumphosphat, α-Tricalciumphosphat, β-Tricalciumphosphat, amorphes Calciumphosphat, Hydroxylapatit, Calcium-defizientes Hydroxylapatit, substituiertes Hydroxylapatit, nichtstöchiometrisches Hydroxylapatit, Nano-Hydroxylapatit, Tetracalciumphosphat, Calciumsulfat, Calciumsulfat Hemihydrat, Calciumsulfat Dihydrat, Calciumoxid, Calciumhydroxid, Calciumcarbonat, Calciumglycerophosphat, Calciumcitrat, Calciumlactat, Calcium acetat, Calciumtartrat, Calciumchlorid, Calciumsilikat, Magnesiumhydrogenphosphat, Trimagnesiumphosphat, Magnesiumdihydrogenphosphat, Magnesiumchlorid, Magnesiumglycero-phosphat, Magnesiumhydroxid, Magnesiumhydroxidcarbonat, Magnesiumoxid (MgO), Magnesium-citrat, Calcium-Magnesiumcarbonat (Dolomit), Magnesiumsilikate.

Bevorzugte Calciumionen-haltige mineralische Zementpulver enthalten zumindest eine der folgenden Verbindungen als Reaktivkomponente: Monocalciumphosphat Monohydrat, Monocalciumphosphat Anhydrit, Dicalciumphosphat Anhydrit, Dicalciumphosphat Dihydrat, Octacalciumphosphat, α-Tricalciumphosphat, β-Tricalciumphosphat, amorphes Calciumphosphat, Hydroxylapatit, Calcium-defizientes Hydroxylapatit, substituiertes Hydroxylapatit, nichtstöchiometrisches Hydroxylapatit, Nano-Hydroxylapatit, Tetracalciumphosphat, Calciumsulfat, Calciumsulfat Hemihydrat, Calciumsulfat Dihydrat, Calciumoxid, Calciumhydroxid, Calciumcarbonat, Calciumglycerophosphat, Calciumcitrat, Calciumlactat, Calciumacetat, Calciumtartrat, Calciumchlorid und Calciumsilikat.

Unter einem Magnesiumionen-haltigen mineralischen Zementpulver wird im Sinne der Erfindung ein mineralisches Zementpulver verstanden, das als Reaktivkomponente zumindest eine der folgenden Verbindungen enthält: Magnesiumhydrogenphosphat, Trimagnesiumphosphat, Magnesiumdihydrogen-phosphat, Magnesiumchlorid, Magnesiumglycero-phosphat, Magnesiumhydroxid, Magnesium-hydroxidcarbonat, Magnesiumoxid (MgO), Magnesiumcitrat oder Magnesiumsilikat.

Zur Abbindung enthalten die Magnesiumionen-haltigen mineralischen Zementpulver weiterhin Hydrogenphosphate, wie z.B. Ammonium-, Natrium- oder Kaliumhydrogenphosphat.

Bevorzugt enthält das mineralische Zementpulver (Calciumionen- oder Magnesiumionenhaltig) zusätzlich Strontiumverbindungen, vorzugsweise Strontiumcarbonate, Strontiumoxide, Strontiumhydroxide und/oder Strontiumphosphate, bevorzugt ausgewählt aus SrHPO₄, Sr₂P₂O₇, Sr₂(PO₄)₂ und Sr₅(PO₄)₃OH.

Die mineralischen Zementpulver werden vorzugsweise vor dem Zusatz zu einer erfindungsgemäßen Zubereitung gemahlen und mittels Sieben oder Sichten fraktioniert.

Bevorzugt werden Pulverpartikel mit einer Größe (maximale Ausdehnung) von 10 nm bis 1000 µm in einer erfindungsgemäßen Zubereitung eingesetzt. Besonders bevorzugt sind in einer erfindungsgemäßen Zubereitung Pulverpartikel mit folgenden Größen enthalten:
- mindestens 10 Gew.-% (bezogen auf die Gesamtmasse der enthaltenen Feststoffe) mit einer Größe von weniger als 5µm,
- mindestens 25 Gew.-% mit einer Größe von 5 µm bis 30µm, und
- mindestens 20 Gew.-% mit einer Größe von mehr als 30 µm.
Vorzugsweise enthält eine erfindungsgemäße Zubereitung mindestens 5 Gew.-% (bezogen auf die Gesamtmasse der enthaltenen Feststoffe) von Pulverpartikeln mit einer Größe von mehr als 250 µm. Besonders bevorzugt bestehen die Pulverpartikel mit einer Größe von mehr als 250 µm aus einem Material, das im Körper schneller resorbiert wird, als der mineralische Zement. Durch die Resorption dieser Pulverpartikel im Implantat werden Poren im Implantat ausgebildet, die das Einwachsen von Knochengewebe ermöglichen.

Bevorzugt beträgt der Massegehalt der in der erfindungsgemäßen Zubereitung enthaltenen Feststoffe (Summe aller in der organischen Trägerflüssigkeit dispergierten und gelösten Feststoffe außer den mindestens zwei Tensiden) bezogen auf die Gesamtmasse der Zubereitung mehr als 75 Gew.-%, vorzugsweise mehr als 80 Gew.-%, vorzugsweise mehr als 85 Gew.-%, besonders bevorzugt mehr als 87,5 Gew.-%. Bevorzugt beträgt der Massegehalt der in der erfindungsgemäßen Zubereitung enthaltenen Feststoffe bezogen auf die Gesamtmasse der Zubereitung zwischen 75 und 95 Gew.-%, vorzugsweise zwischen 80 und 95 Gew.-%, besonders bevorzugt zwischen 85 und 95 Gew.%.

Bevorzugt beträgt der Massegehalt des in der erfindungsgemäßen Zubereitung enthaltenen mineralischen Zementpulvers bezogen auf die insgesamt in der erfindungsgemäßen Zubereitung enthaltenen Feststoffe (Summe aller in der organischen Trägerflüssigkeit dispergierten und gelösten Feststoffe außer den mindestens zwei Tensiden) mehr als 25 Gew.-%, besonders bevorzugt mehr als 50 Gew.-%, weiter bevorzugt 50 bis 80 Gew.-%. Das mineralische Zementpulver ist dabei die reaktive Komponente, die Gesamtmasse der Feststoffe umfasst zusätzlich die enthaltenen Füllstoffe.

Die erfindungsgemäßen Zubereitungen enthalten mindestens eine organische Trägerflüssigkeit, in der das mineralische Zementpulver dispergiert ist. Die organische Trägerflüssigkeit ist so ausgewählt, dass sie selbst nicht mit dem mineralischen Zementpulver reagiert. Grundsätzlich sind schwer wasserlösliche Trägerflüssigkeiten geeignet. Unter schwer wasserlöslich werden im Sinne der Erfindung Verbindungen verstanden, deren maximale Löslichkeit in Wasser 1,0 mol/l, bevorzugt 0,1 mol/l, beträgt. Erfindungsgemäß sind schwer wasserlösliche Trägerflüssigkeiten. Bevorzugt sind hydrophobe Trägerflüssigkeiten.
In erfindungsgemäßen Zubereitungen eingesetzte Trägerflüssigkeiten sind vorzugsweise bioverträglich.

Bevorzugte schwer wasserlösliche Trägerflüssigkeiten sind ausgewählt aus Glycerintriacetat, Glycerintributyrat, Glycerintrioleat, kurzkettigen Triglyceriden und mittelkettigen Triglyceriden. Erfindungsgemäße Trägerflüssigkeiten sind kurz- oder mittelkettige Triglyceride. Unter kurzkettigen Fettsäureverbindungen werden Verbindungen von Fettsäuren einer Länge von jeweils 2 bis 5 Kohlenstoffatomen verstanden. Unter mittelkettigen Fettsäureverbindungen werden Verbindungen von Fettsäuren einer Länge von jeweils 6 bis 14 Kohlenstoffatomen verstanden.
Erfindungsgemäß sind Triglyceride mit enthaltenen Fettsäuren, die durchschnittlich weniger als 14 C-Atome aufweisen.
Bevorzugte erfindungsgemäße Zubereitungen enthalten mindestens eine wasserlösliche und mindestens eine schwer wasserlösliche organische Trägerflüssigkeit. Dadurch kann vorteilhaft die Abbindegeschwindigkeit der erfindungsgemäßen Zubereitung in Wasser beeinflusst werden. Ein weiterer Vorteil der Kombination aus mindestens einer wasserlöslichen und mindestens einer schwer wasserlöslichen Trägerflüssigkeit ist es, dass der Feststoffgehalt der Paste erhöht werden kann. Verbindungen mit einer maximalen Löslichkeit in Wasser von mehr als 1 mol/l (vorzugsweise mehr als 3 mol/l) werden hierin als wasserlöslich bezeichnet. Beim Einsatz wasserlöslicher Trägerflüssigkeiten ist für die Lagerung der erfindungsgemäßen Zubereitung eine wasserdichte Verpackung erforderlich, um das Aushärten der Zubereitung an der Umgebungsluft zu verhindern.
Bevorzugt beträgt der Gewichtsanteil der organischen Trägerflüssigkeit bezogen auf die Gesamtmasse der Zubereitung 5 bis 25 Gew.-%, vorzugsweise 5 bis 20 Gew.-%, vorzugsweise 5 bis 15 Gew.-%, vorzugsweise 5 bis 12,5 Gew.-%.
Vorzugsweise enthält eine erfindungsgemäße Zubereitung weiterhin mindestens einen Abbindebeschleuniger. Dadurch werden vorteilhaft die Abbindezeit und der pH-Wert-Verlauf bei der Aushärtung der erfindungsgemäßen Zubereitung eingestellt. Bevorzugte Abbindebeschleuniger sind Phosphatsalze, organische Säuren oder Salze organischer Säuren. Bevorzugt sind natrium- und/oder kaliumionenhaltige Phosphate oder natrium- und/oder kaliumionenhaltige Salze organischer Säuren. Besonders bevorzugt sind kaliumionenhaltige Phosphate (vorzugsweise Kaliumphosphate, insbesondere Kaliumdihydrogenphosphat und di-Kaliumhydrogenphosphat). Mit kaliumionenhaltigen Phosphaten konnte eine besonders vorteilhafte Abbindekinetik, besonders in Kombination mit nichtionischen Tensiden (ganz besonders gut in Kombination mit nichtionischen und anionischen Tensiden), erzielt werden. Eine besonders bevorzugte erfindungsgemäße Zubereitung enthält mindestens ein ionisches (anionisch) und mindestens ein nicht-ionisches Tensid mit einem mittelkettigen Triglycerid als organische Trägerflüssigkeit sowie mindestens ein kaliumionenhaltiges Phosphat (vorzugsweise di-Kaliumhydrogenphosphat) als Abbindebeschleuniger. Dadurch wird vorteilhaft eine lange Haltbarkeit der Zubereitung erzielt.
Der Abbindebeschleuniger ist bevorzugt in einem Massenanteil (in Bezug auf die Masse des mineralischen Zementpulvers) von 0,1 bis 5 %, besonders bevorzugt 0,2 bis 4 %, ganz besonders bevorzugt 0,5 bis 3,5 % in der erfindungsgemäßen Zubereitung enthalten.
Die erfindungsgemäßen Zubereitungen enthalten mindestens zwei unterschiedliche Tenside, ausgewählt aus mindestens zwei der Gruppen der anionischen, kationischen, amphoteren und nichtionischen Tenside. Erfindungsgemäß sind mindestens ein nichtionisches und mindestens ein anionisches Tensid in einer erfindungsgemäßen Zubereitung enthalten, wobei die Gesamtmasse der nichtionischen Tenside vorzugsweise mindestens das Doppelte der Gesamtmasse der anionischen Tenside beträgt.
Die Tenside erleichtern die Einarbeitung der Feststoffe in die organische Trägerflüssigkeit. Besonders bevorzugt sind Kombinationen aus mindestens einem hydrophilen Tensid und mindestens einem lipophilen Tensid. Ganz besonders bevorzugt ist in einer erfindungsgemäßen Zubereitung mindestens ein Tensid mit einem HLB-Wert (Hydophil-Lipophil-Balance, Masseverhältnis zwischen dem polaren und dem unpolaren Teil eines Tensids) von mehr als 8 (hydrophiles Tensid) und mindestens ein Tensid mit einem HLB-Wert von weniger als 5 (lipophiles Tensid) enthalten.
Bevorzugt sind Tenside in einer erfindungsgemäßen Zubereitung zu einem Masseanteil von insgesamt (Summe der Masse aller enthaltenen Tenside bezogen auf die Gesamtmasse der Zubereitung) 0,1 bis 10 Gew.-%, vorzugsweise 0,5 Gew.-% bis 5 Gew.-%, bevorzugt 1 Gew.-% bis 3,5 Gew.-%, enthalten.
Ist in einer erfindungsgemäßen Zubereitung mindestens ein nichtionisches Tensid enthalten, so liegen das nichtionische Tensid (Gesamtmasse der nichtionischen Tenside) und die organische Trägerflüssigkeit in einem Gewichtsverhältnis von 1:1 bis 1:25, vorzugsweise 1:5 bis 1:20, vor.

Erfindungsgemäß enthält eine erfindungsgemäße Zubereitung mindestens ein anionisches und mindestens ein nichtionisches Tensid, ausgewählt aus
- einen ethoxylierten Fettalkohol, mindestens eine ethoxylierte Fettsäure, mindestens einen ethoxylierten Sorbitanfettsäureester (insbesondere Polysorbat 80), mindestens ein ethoxyliertes Fett oder mindestens ein ethoxyliertes Öl (insbesondere polyethoxyliertes Castoröl) und
- mindestens einen mit Zitronensäure, Schwefelsäure oder Phosphorsäure veresterten Fettalkohol, mindestens ein mit Zitronensäure, Schwefelsäure oder Phosphorsäure verestertes Mono- oder Diglycerid oder Salze davon oder mindestens eine mit Zitronensäure, Schwefelsäure oder Phosphorsäure veresterte Fettsäure oder Salze davon.

Vorzugsweise enthalten erfindungsgemäße Zubereitungen mindestens einen Füllstoff. Unter Füllstoffen werden im Sinne der Erfindung Substanzen verstanden, die bei der Herstellung der erfindungsgemäßen Zubereitung nicht bereits im mineralischen Zementpulver enthalten sind, sondern die erst nach der Dispersion des mineralischen Zementpulvers in der organischen Trägerflüssigkeit in Gegenwart der mindestens zwei Tenside zugegeben werden. Die Füllstoffe werden dabei zur Einstellung der Eigenschaften (insbesondere Fließfähigkeit, Resorbierbarkeit oder Röntgenkontrast) der erfindungsgemäßen Zubereitung zugegeben. Bevorzugte Füllstoffe sind ausgewählt aus Strontiumcarbonat, Strontiumhydrogenphosphat, Strontiumphosphat, Glaskeramiken (vorzugsweise resorbierbare Glaskeramiken, insbesondere Glaskeramiken umfassend SiO₂, Na₂O, CaO und P₂O₅ und ggf. zusätzlich enthaltend MgO und/oder K₂O), Calciumcarbonat, Eisenoxiden, Siliciumdioxiden, Bariumsulfat, Glycerinstearat, gefälltem nanokristallinen Hydroxylapatit, calciumdefizienten Hydroxylapatit und Tricalciumphosphat, insbesondere beta-Tricalciumphosphat. Die Füllstoffe liegen in partikulärer Form vor.

Als Füllstoffe sind vorteilhaft auch mineralische oder organische Fasern geeignet. Beispiele sind organische Fasern auf der Basis resorbierbarer Polymere, die sich z. B. aus den resorbierbaren Nahtmaterialien ableiten. Solche Fasern können die Bruchfestigkeit des ausgehärteten Zements erhöhen. Konventionelle mineralische Knochenzemente mit enthaltenen polymeren Kurzfasern sind aus der Literatur bekannt (insbesondere Norian screwable).

Bevorzugt sind erfindungsgemäße Zubereitungen, die als Füllstoffe mineralische Kurzfasern enthalten, insbesondere keramische und glasartige Kurzfasern. Beispiele für solche Fasern sind Fasern auf der Basis von Wollastonit und (insbesondere biolösliche) Glasfasern, umfassend ebenfalls Kieselgelfasern und Kieselsäurefasern. Beispiele für geeignete Glasfasern sind insbesondere Kurzstapelfasern auf der Basis von Kalk-Alkali-Gläsern, sogenannten Biogläsern - insbesondere Bioglass der Zusammensetzung 45S5 (45S5 bezeichnet mit "45" den prozentualen Anteil an SiO₂ und mit "5" das Verhältnis von CaO zu P₂O₅). Zahlreiche andere Formulierungen sind in der wissenschaftlichen Literatur beschrieben.

Ebenfalls bevorzugt sind Kieselsäurefasern, z. B. Belcotex Fasern der Fa. Belchem, Freiberg mit mittleren Faserdurchmessern <10µm und Faserlängen von <5mm. Besonders bevorzugt sind Fasern ohne Aluminiumanteil, insbesondere solche, die außer SiO₂ und ggf. Phosphat ausschließlich Alkali- und Erdalkaliionen enthalten.

Die mineralischen Fasern sind in erfindungsgemäßen Zubereitungen in einem Gehalt von 0,1 bis 30 Gew.-% enthalten, bevorzugt zwischen 1 und 20 Gew.-%. Sie haben einen hohen Einfluss auf das Bruchverhalten der ausgehärteten Formkörper, wie aus den Ergebnissen in Beispiel 6 hervorgeht. Während die Druckfestigkeit nur leicht ansteigt, brechen die verstärkten Formkörper nicht katastrophal, sondern können auch nach Überschreiten der maximalen Druckkraft noch Last aufnehmen. Für Implantatanwendungen ist dieses Deformationsverhalten besonders vorteilhaft, da auch ein angebrochenes Implantat durch den Regenerationsprozess im Knochen wieder integriert werden kann.
Bevorzugte Fasern haben einen mittleren Durchmesser von 1µm bis 300µm, bevorzugt 3µm bis 100µm.

Substanzen, die mit Wasser unter Bildung eines schwerlöslichen Festkörpers reagieren (und damit die identische chemische Zusammensetzung wie mineralische Zementpulver im Sinne der Erfindung haben), können zusätzlich neben dem enthaltenen mineralischen Zementpulver in der erfindungsgemäßen Zubereitung als Füllstoff enthalten sein. Für die Bereitstellung des mineralischen Zementpulvers werden dessen Bestandteile unter Vermahlen miteinander vermischt. Die Füllstoffe, die zur erfindungsgemäßen Zubereitung zugegeben werden, werden nicht gemeinsam mit den Bestandteilen des mineralischen Zementpulvers vermahlen. Füllstoffe werden erst nach Vermischung des mineralischen Zementpulvers mit der organischen Trägerflüssigkeit zur erfindungsgemäßen Zubereitung zugegeben. Auf diese Weise werden durch die Zusammensetzung der Partikelgrößen (unter der Partikelgröße wird hierein die maximale Ausdehnung des Partikels verstanden, bei runden Partikeln entspricht dies dem Partikeldurchmesser) der Füllstoffe u. a. die rheologischen Eigenschaften der erfindungsgemäßen Zubereitung beeinflusst. Durch den Zusatz von Füllstoffen können zudem die mechanischen Eigenschaften der erfindungsgemäßen Zubereitungen und der daraus erhältlichen soliden Formkörper beeinflusst werden.

Bevorzugt enthalten erfindungsgemäße Zubereitungen zu mehr als 5 Gew.-% (in Bezug auf die Gesamtmasse der in der Zubereitung enthaltenen Feststoffe) partikuläre Füllstoffe mit einer Partikelgröße von weniger als 10 µm. Diese sind bevorzugt ausgewählt aus Strontiumcarbonat, Calciumcarbonat, gefälltem nanokristallinen Hydroxylapatit und calciumdefizientem Hydroxylapatit.
Bevorzugt enthalten erfindungsgemäße Zubereitungen zu mehr als 20 Gew.-% partikuläre Füllstoffe mit einer Partikelgröße von mehr als 50 µm, vorzugsweise mehr als 100 µm. Die Partikelgröße der Füllstoffe beträgt dabei vorzugsweise maximal 5 mm. Füllstoffe mit einer Partikelgröße von mehr als 50 µm sind bevorzugt ausgewählt aus α-Tricalciumphosphat, β-Tricalciumphosphat, Calciumhydrogenphosphat, Glaskeramiken (vorzugsweise resorbierbare Glaskeramiken, insbesondere Glaskeramiken umfassend SiO₂, Na₂O, CaO und P₂O₅ und ggf. zusätzlich enthaltend MgO und/oder K₂O), gesintertem Hydroxylapatit, Calciumcarbonat, gesintertem oder gebranntem Magnesiumphosphat, Calciummagnesiumphosphat, Magnesiumammoniumphosphat (als Hydrat oder wasserfrei).

Vorzugsweise enthalten die erfindungsgemäßen Zubereitungen mindestens einen polymeren Hilfsstoff, bevorzugt ausgewählt aus Kollagen, Gelatine und deren Derivaten, Stärke und deren Derivaten (bevorzugt Hydroxyethylstärke, Carboxymethylstärke), Cellulosederivaten, Chitin, Chitosan und deren Derivaten, Polyvinylalkohol, Polyvinylpyrrolidon, Polyacrylsäure und deren Derivaten (insbesondere Polycarboxylat-Ether), Polymethacrylsäure und deren Derivaten (insbesondere Copolymere von Methacrylsäure mit Methylmethacrylat, Ethylacrylat und/oder Methylacrylat), Polymethylmethacrylat, Polystyrol und Copolymeren mit Monomeren von Methylmethacrylat und Styrol.

Vorzugsweise enthalten die erfindungsgemäßen Zubereitungen wasserlösliche partikuläre Füllstoffe aus mineralischen oder organischen Substanzen. Durch den Einsatz wasserlöslicher Partikel kann vorteilhaft die Porosität des bei Aushärtung mit Wasser gebildeten Feststoffs eingestellt werden. Wasserlösliche Füllstoffe weisen vorzugsweise eine Partikelgröße von 50 µm bis 2000 µm, bevorzugt von 100 µm bis 1000 µm, auf. Wasserlösliche Füllstoffe sind in der erfindungsgemäßen Zubereitung bevorzugt zu 5 bis 90 Vol.-%, vorzugsweise 10 bis 80 Vol.-% enthalten (bezogen auf das Gesamtvolumen der erfindungsgemäßen Zubereitung). Bevorzugte wasserlösliche Füllstoffe sind ausgewählt aus Zuckern (vorzugsweise Saccharose), Zuckeralkoholen (vorzugsweise Sorbit, Xylit, Mannit), wasserlöslichen Salzen (vorzugsweise Natriumchlorid, Natriumcarbonat oder Calciumchlorid). Bevorzugt werden die wasserlöslichen Füllstoffe in Form von Granulaten eingesetzt.
Vorzugsweise enthalten erfindungsgemäße Zubereitungen mindestens einen pharmazeutischen Wirkstoff, vorzugsweise Wirkstoffe mit wachstumsstimulierender oder antimikrobieller Wirkung. Bevorzugte Wirkstoffe sind ausgewählt aus Antibiotika, Antiseptika, antimikrobiellen Peptiden, antiresorptive Wirkstoffe (vorzugsweise Bisphosphonate, Cortikoide, Fluoride, Protonenpumpen-Inhibitoren), knochenwachstumsstimulierende Wirkstoffe (vorzugsweise Wachstumsfaktoren, Vitamine, Hormone, Morphogene, davon bevorzugt knochenmorphogenetische Proteine).

Die erfindungsgemäßen Zubereitungen sind vorzugsweise als Ein-Pastensystem formuliert. Nach einer Ausgestaltung sind die erfindungsgemäßen Zubereitungen als Zwei-Komponentensystem mit einer wasserfreien Paste und einer wasserenthaltenden Komponente formuliert.
Die wasserfreie Paste enthält dazu die erfindungsgemäße Zubereitung in einer der oben beschriebenen Ausführungsformen.

Die wasserhaltige Komponente enthält dazu eine wässrige Lösung, eine wässrige Dispersion oder reines Wasser.
Überraschend hat sich herausgestellt, dass sich die erfindungsgemäßen Zubereitungen mit einem sehr breiten Spektrum an wasserhaltigen Komponenten hervorragend und makroskopisch homogen mischen lassen. Eine exzellente Mischung wird auch erreicht, wenn die wasserhaltige Komponente als Lösung von wasserlöslichen Polymeren, gelösten Wirkstoffen wie z. B. Antibiotika oder als wässrige Dispersion von Feststoffen wie z. B. dispergierten Knochenmineralien oder deren synthetischen Analogen besteht. Ebenfalls wurden sehr gute Mischungen erreicht, wenn als wasserhaltige Komponente Blut, Serum, Knochenmarksaspirat oder plättchenreiches Plasma verwendet wurden. Die erfindungsgemäße Zubereitung lässt sich entsprechend vielseitig kombinieren. Besonders vorteilhaft ist die Kombination der erfindungsgemäßen Zubereitung mit einer wässrigen Lösung oder Dispersion bei Verwendung einer Doppelkammerkartusche mit einem fest vorgegebenen Mischungsverhältnis der beiden zu mischenden Komponenten, wobei die Mischung bei der Austragung erfolgt. Bevorzugt ist die Mischung der erfindungsgemäßen Zubereitung: wässrige Komponente im Verhältnis ≥2:1 bis 4:1, besonders bevorzugt im Verhältnis ≥ 4:1 bis 10:1.

Vorteile des Zwei-komponentensystems sind eine einfache Einmischung von wasserlöslichen Wirkstoffen und biologischen Komponenten, die erst vor Ort mit der erfindungsgemäßen Zubereitung kombiniert werden sollen, weiterhin die Vermeidung von Inkompatibilitäten während der Herstellung und Lagerung und die Kontrolle der Abbindebedingungen durch dosierte Zugabe der wässrigen Komponente. Bei großen Mengen an implantiertem Material ist eine Vermischung mit einer wässrigen Komponente zuverlässiger und führt zu einer schnelleren Aushärtung als die passive Aushärtung beim Ein-Pastensystem.

Zur Erfindung gehört auch die Verwendung einer erfindungsgemäßen Zubereitung zur Herstellung als Material zur Behandlung von Knochendefekten oder zum Knochenaufbau, zur Befestigung von Knochenimplantaten oder zur Herstellung von implantierbaren Wirkstoffträgern. Vorteilhaft ist hier die Druckfestigkeit von mehr als 25 MPa.

Die Erfindung umfasst auch Verfahren zur Herstellung eines Implantats, vorzugsweise eines Knochenimplantats. Dabei wird eine erfindungsgemäße Zubereitung mit einer Wasser enthaltenen Zubereitung kontaktiert. Durch die Reaktion des Wassers mit dem mineralischen Zementpulver wird eine Abbindereaktion ausgelöst und ein fester Körper gebildet. Die Wasser enthaltende Zubereitung ist dabei entweder reines Wasser, eine wässrige Lösung oder eine Dispersion von Feststoffen in einem wässrigen Lösungsmittel (wässrige Paste). Für die Auslösung der Abbindereaktion genügt das Einbringen (Einlegen) der erfindungsgemäßen Zubereitung in Wasser oder eine wässrige Lösung. Die Auslösung der Abbindereaktion einer erfindungsgemäßen Zubereitung kann auch nach der Implantation erfolgen, wobei die erfindungsgemäße Zubereitung durch das in der Umgebung des Implantats vorhandene Wasser aushärtet. Wird als Wasser enthaltende Zubereitung eine wässrige Paste eingesetzt, so wird die erfindungsgemäße Zubereitung mit der Paste (vorzugsweise unter Rühren) vermengt und dadurch die mineralischen Zementbestandteile mit dem Wasser in Kontakt gebracht. Dies erfolgt vor der Implantation in den Körper. Dafür liegt die erfindungsgemäße Zubereitung vorzugsweise in einem geeigneten Mischsystem, insbesondere einer Zwei-Kammer-Spritze vor, wobei in einer Kammer die erfindungsgemäße Zubereitung und in der anderen Kammer die Wasser enthaltende Zubereitung enthalten ist.

Bevorzugt werden dreidimensionale Formkörper aus einer erfindungsgemäßen Zubereitung hergestellt. Aufgrund des hohen Feststoffgehalts der erfindungsgemäßen Zubereitung sind Formkörper aus den erfindungsgemäßen Zubereitungen auch im unausgehärteten Zustand nach Verpackung über lange Zeiträume ohne Veränderung der Dimensionen (Zusammensinken) stabil.

Durch die vorteilhaften Fließeigenschaften der Zubereitung eignet sich diese für die Anwendung in Verfahren des Dreidimensionalen Plottens (3D Plotten). Dabei wird aus der Zubereitung mittels Extrusion ein dreidimensionaler Formkörper aufgebaut. Auch hier ist es besonders vorteilhaft, dass die erfindungsgemäße Zubereitung sowohl gut dosierbar ist (hohe Strukturviskosität) und gleichzeitig einen hohen Feststoffgehalt aufweist, so dass die Formkörper auch im unausgehärteten Zustand nicht zusammensinken und sich dadurch die zuvor definierten Dimensionen verändern. Nach Einbringen des dreidimensionalen Formkörpers in Wasser oder eine wässrige Lösung härtet der Formkörper aus. Auf diese Weise ist es vorteilhaft möglich, ein auf den Patienten maßgeschneidertes Implantat bereitzustellen.

Die Erfindung umfasst auch feste Formkörper, die nach Kontaktieren einer erfindungsgemäßen Zubereitung mit einer Wasser enthaltenden Zubereitung (vorzugsweise Wasser, einer wässrigen Lösung oder einer Dispersion von Feststoffen in einem wässrigen Lösungsmittel) erhältlich sind. Die Formkörper sind vorzugsweise offenporig und weisen ein interkonnektiertes Porensystem auf. Die Poren weisen dabei vorzugsweise einen mittleren Durchmesser von > 50 µm und < 1000µm auf (der mittlere Durchmesser ist dabei die über alle Poren gemittelte maximale Ausdehnung der Poren des gesamten Formkörpers). Es ist mit dem Verfahren des 3D Plots vorteilhaft möglich, das Porensystem des Formkörpers gezielt einzustellen und dadurch wahlweise gleichmäßige Porensysteme zu erzeugen oder Poren in Vorzugsrichtungen anzuordnen.

Bevorzugt weisen Formkörper, welche Poren mit einem maximalen Durchmesser von 50 µm enthalten (solide makroporenfreie Formkörper, diese sind durch Aushärten einer erfindungsgemäßen Zubereitung in wässriger Umgebung erhältlich), eine Druckfestigkeit von mehr als 20 MPa auf. Der Formkörper, der nach Aushärten einer erfindungsgemäßen Zubereitung gebildet wird, weist vorzugsweise ein Ca/PO₄-Verhältnis von mehr als 1,35, vorzugsweise mindestens 1,5, vorzugsweise mindestens 1,6 auf.
Die Erfindung umfasst auch ein Verfahren zur Herstellung einer erfindungsgemäßen Zubereitung. In dem Verfahren wird
a) mineralisches Zementpulver oder einzelne Bestandteile des mineralischen Zementpulvers sukzessive durch Vermahlung in der organischen Trägerflüssigkeit in Gegenwart der mindestens zwei Tenside dispergiert,
   wobei die organische Trägerflüssigkeit eine schwer wasserlösliche Trägerflüssigkeit ist, wobei die schwer wasserlösliche Trägerflüssigkeit ausgewählt ist aus kurz- oder mittelkettigen Triglyceriden, wobei kurzkettige Triglyceride Verbindungen von Fettsäuren einer Länge von jeweils 2 bis 5 Kohlenstoffatomen sind, wobei mittelkettige Triglyceride Verbindungen von Fettsäuren einer Länge von jeweils 6 bis 14 Kohlenstoffatomen sind,
   dadurch gekennzeichnet,
   dass das Gewichtsverhältnis der insgesamt in der Zubereitung enthaltenen Feststoffe zur Summe des Gewichts der organischen Trägerflüssigkeit und der mindestens zwei Tenside mehr als 5 beträgt, wenn das Zementpulver Calciumionen-haltige Verbindungen und keine Magnesiumionen¬haltigen Verbindungen als Reaktivkomponente enthält, oder
   mehr als 6 beträgt, wenn das Zementpulver Magnesiumionen-haltige oder Magnesiumionen- und Calciumionen-haltige Verbindungen als Reaktivkomponenten enthält, und
   dass mindestens ein anionisches und mindestens ein nichtionisches Tensid enthalten ist, wobei das mindestens eine anionisches und mindestens eine nichtionische Tensid mindestens ein ethoxylierter Fettalkohol, mindestens eine ethoxylierte Fettsäure, mindestens ein ethoxylierter Sorbitanfettsäureester, mindestens ein ethoxyliertes Fett oder mindestens ein ethoxyliertes Öl und mindestens ein mit Zitronensäure, Schwefelsäure oder Phosphorsäure veresterter Fettalkohol, mindestens ein mit Zitronensäure, Schwefelsäure oder Phosphorsäure verestertes Mono- oder Diglycerid oder Salz davon oder mindestens eine mit Zitronensäure, Schwefelsäure oder Phosphorsäure veresterte Fettsäure oder Salze davon sind.
b) Bevorzugt werden anschließend weitere Bestandteile des mineralischen Zementpulvers und/oder Füllstoffe, jeweils mit einer Partikelgröße von weniger als 10 µm, unter ständigem Vermahlen zugegeben.
c) Bevorzugt werden anschließend Füllstoffe mit einer Partikelgröße von mehr als 50 µm zugegeben. Die Zubereitung wird weiterhin vermischt (vorzugsweise durch Rühren oder Kneten), bis eine homogene Paste gebildet ist. Der Verfahrensschritt c) wird bevorzugt nicht unter Vermahlen durchgeführt.

Mit diesem Verfahren ist es vorteilhaft möglich, erfindungsgemäße Zubereitungen mit einem Feststoffgehalt von mehr als 85 Gew.-% (bezogen auf die Gesamtmasse der Zubereitung) herzustellen. Dabei werden im ersten Verfahrensschritt zunächst die mit Wasser reaktiven Bestandteile des mineralischen Zementpulvers vermahlen und in der organischen Trägerflüssigkeit in Anwesenheit von mindestens zwei Tensiden dispergiert. Die mineralischen Zementpulver werden dafür zuvor mit bekannten Verfahren, vorzugsweise durch Vermahlen der enthaltenen mit Wasser reaktiven Bestandteile, hergestellt. In einem zweiten Verfahrensschritt werden bevorzugt sehr feinteilige Partikel mit einer maximalen Ausdehnung von weniger als 10 µm (z.B. als Kristallisationskeime) unter Vermahlen zugegeben, wodurch die Viskosität der Zubereitung stark erhöht wird. In einem dritten Verfahrensschritt wird v.a. der Feststoffgehalt der Zubereitung durch die Zugabe großer Partikel mit einer maximalen Ausdehnung von mehr als 50 µm eingestellt. Aus diesem Grund wird beim Zusatz der groben Partikel auch kein Mahlverfahren eingesetzt, sondern die Partikel lediglich unter die im ersten oder zweiten Verfahrensschritt gebildete Zubereitung untergemischt.

In dem Verfahren werden im ersten Verfahrensschritt zunächst die Tenside zur Trägerflüssigkeit gegeben, bis eine homogene Flüssigkeit gebildet ist. Anschließend wird das mineralische Zementpulver oder dessen Bestandteile portionsweise unter Vermahlen zugegeben, bis eine homogene Paste entstanden ist.

Mit der Erfindung werden leicht verarbeitbare und lagerstabile Zubereitungen zur Herstellung von Implantaten angegeben, mit denen Implantate mit hohen Druckfestigkeiten hergestellt werden können. Aufgrund der wesentlichen Abwesenheit von Wasser in der Zubereitung härtet diese erst nach Einbringen in Wasser oder eine wässrige Lösung aus. Durch den Einsatz mehrerer Tenside wird die Einarbeitung der Feststoffe in die organische Trägerflüssigkeit erleichtert.

Aufgrund des Einsatzes eines besonders hohen Gewichtsverhältnisses von den insgesamt in der Zubereitung enthaltenen Feststoffen zur Summe des Gewichts der organischen Trägerflüssigkeit und der mindestens zwei Tenside von mehr als 5 (für Calciumionen-haltige mineralische Zementpulver) und von mehr als 6 (für Magnesium-haltige mineralische Zementpulver) sind vorteilhaft Formkörper (Implantate) mit einer besonders hohen Druckfestigkeit erhältlich. Es hat sich überraschenderweise herausgestellt, dass die Druckfestigkeit der erhältlichen Formkörper beim Überschreiten der vorgenannten Gewichtsverhältnisse in der Zubereitung sprunghaft ansteigt.

Bei der Betrachtung der Gehalte an dispergierten Feststoffen ist zu berücksichtigen, dass eine Steigerung des Feststoffanteils in einer Dispersion auch bei nominal kleinen Unterschieden einen sehr großen Effekt haben kann. Eine Steigerung von z. B. 80% auf 85% Feststoffgehalt bedeutet, dass 25% weniger Flüssigkeit zur Dispergierung der Feststoffe bereitstehen (bezogen auf 100g: 15g Trägerflüssigkeit statt 20g). Dies gilt umso mehr, als ein Teil der Trägerflüssigkeit aus Tensiden besteht, die als oberflächenaktive Substanzen an den mineralischen Partikeln anhaften und daher nur bedingt als Bestandteil der Trägerflüssigkeit zur Verfügung stehen. Beim Amphisol A (in den Beispielen) handelt es sich zudem um einen Feststoff, der nur unter Erwärmung in der Trägerflüssigkeit löslich ist und beim Abkühlen wieder ausfällt. Die beschriebene Erhöhung des Feststoffgehalts ist daher als erhebliche Änderung der Zusammensetzung im Vergleich zum Stand der Technik anzusehen.

Der höchste im Stand der Technik beschriebene Feststoffgehalt für Einkomponenten Pasten-Calciumphosphat-Zemente ist in Tabelle 1 als Vergleichsbeispiel angegeben. Bei einem Gesamtfeststoffgehalt von 81,2% und einem entsprechenden Gewichtsverhältnis von Feststoff zu Trägerflüssigkeit von 4,32:1 ist der Zement aus dem Vergleichsbeispiel in seiner Konsistenz und in seinen Verarbeitungseigenschaften den erfindungsgemäßen Präparationen 4 und 5 in Tabelle 1 vergleichbar. Die erfindungsgemäßen Präparationen 4 und 5 in Tabelle 1 weisen im Unterschied zum Vergleichsbeispiel aber einen Feststoffgehalt von jeweils 87,84% auf, entsprechend einem Gewichtsverhältnis von Feststoff zu Trägerflüssigkeit von 7,22:1. Dieses stark erhöhte Verhältnis von Feststoff zu Trägerflüssigkeit bewirkt bei vergleichbarer Pastenkonsistenz eine signifikante Verbesserung der Pastenstabilität gegen Sedimentation oder Separation und eine Verdoppelung der Druckfestigkeit der aus diesen Präparationen hergestellten Prüfkörper.

Überraschend wurde ebenfalls gefunden, dass die erfindungsgemäßen Zubereitungen - trotz der hohen Beladung mit Feststoffen und der intensiven Vermahlung/Vermischung, die zu einer sehr dichten und praktisch porenfreien Dispersion führt - nach Einbringung in eine wässrige Lösung - in den Standarduntersuchungen wurde eine 0,9%ige Kochsalzlösung verwendet - spontan aushärten. In den entsprechenden Tests wurden die hergestellten erfindungsgemäßen pastösen Präparationen ohne weitere Vermischung mit Wasser oder wässrigen Lösungen in quaderförmige Formen der Abmessung 6x6x12mm eingefüllt, so dass die jeweilige Form vollständig ausgefüllt war. Die gefüllten Formen wurden anschließend in die 0,9%ige Kochsalzlösung eingetaucht und bei 37°C für 24 Stunden inkubiert. Nach dieser Zeit war die Paste zu Formkörpern ausgehärtet und die Formkörper konnten als solide Blöcke entnommen werden. Zur weiteren und vollständigen Aushärtung wurden die entnommenen Formkörper für weitere 72 Stunden bei 37°C inkubiert. Die Druckfestigkeitsuntersuchungen wurden an so hergestellten Formkörpern durchgeführt. Die Messung der Druckfestigkeit erfolgte an aufrecht stehenden Formkörpern entlang deren längster Achse nach der Inkubation an noch feuchten Formkörpern.

Dieses Verhalten der erfindungsgemäßen Zubereitungen war nicht zu erwarten, da davon auszugehen war, dass eine deutliche Steigerung des Feststoffgehalts nur über eine starke Verdichtung der Partikel in der pastösen Dispersion zu erreichen ist. Dies sollte zu einer entsprechenden Verkleinerung der ölgefüllten Poren führen, wodurch die Verdrängung des Öls durch eine wässrige Lösung (die für die Abbindereaktion erforderlich ist) stark behindert wird. Die Untersuchungsergebnisse zeigen allerdings überraschend, dass auch in einer Schichtdicke von >6mm ein Zutritt von Wasser innerhalb von <24h erreicht wird und ohne mechanische Vermischung eine Durchhärtung der Formkörper erfolgt.

Versuche mit verschiedenen Tensiden und Tensidkombinationen ergaben zudem, dass das beschriebene Aushärtungsverhalten keineswegs selbstverständlich oder vorhersehbar war. So konnten unter Verwendung von ausschließlich anionischen Tensiden zwar ebenfalls sehr hohe Feststoffgehalte in den Pasten realisiert werden, allerdings erfolgte bei diesen Präparationen auch nach sehr langer Inkubation in wässrigen Lösungen keine Durchhärtung des Materials. Bei der Verwendung von ausschließlich nichtionischen Tensiden zeigten die Pasten nach Einbringung in wässrige Lösungen eine starke Zerfallsneigung, so dass solche Präparationen für die klinische Anwendung nur eingeschränkt anwendbar wären.

Die erfindungsgemäßen Zubereitungen sind daher zusätzlich darüber definiert, dass sie ohne weitere Vermischung nach Einbringung in wässrige Lösungen auch in dickeren Schichten durchhärten (>3mm innerhalb von 24h bei 37°C formstabil werden).

Die erfindungsgemäßen Zubereitungen (bzw. spezielle Ausführungsformen) sind weiterhin darüber definiert, dass sie als Basismaterial ohne porenbildende Zusätze und ohne Wirkstoffe, nach vollständiger Aushärtung, ohne aktive Mischung mit einer wässrigen Lösung, eine Druckfestigkeit von >20MPa erreichen.

Ferner wurde festgestellt, dass erfindungsgemäße Zubereitungen durch die enthaltene Kombination aus mehreren Tensiden (mindestens einem nichtionischen und einem anionischen Tensid) und dem genannten Gewichtsverhältnis besonders vorteilhafte Kohäsionseigenschaften aufweisen. Es konnte gezeigt werden, dass die erfindungsgemäßen Zubereitungen weniger zur Fest-Flüssig-Separation neigen als bekannte pastöse Zementzubereitungen. Dadurch sind die erfindungsgemäßen Zubereitungen deutlich lagerstabiler. Erfindungsgemäße Zubereitungen setzen bei Inkubation in einer wässrigen Flüssigkeit (beispielsweise 0,9% NaCl oder simulierter Körperflüssigkeit) lediglich maximal etwa 1 Gew.-% filtrierbare Partikel frei. Dadurch bleiben aus der pastösen Zubereitung gefertigte Formkörper auch nach Einbringen in eine wässrige Flüssigkeit sehr gut in ihrer Form, es werden kaum Partikel freigesetzt. Es ist dadurch sichergestellt, dass erfindungsgemäße Zubereitungen nach Einbringung in den Körper (vor, während und nach der Abbindereaktion) nahezu keine Partikel freisetzen. Dieses Problem tritt bei bekannten Zementen häufig auf und wird für zementbedingte Entzündungsreaktionen verantwortlich gemacht.

Erfindungsgemäße Zubereitungen sind sehr gut dosierbar und sind strukturviskos. Auch bei hohem Feststoffanteil ist so eine zuverlässige Dosierung ohne sehr hohen Kraftaufwand sichergestellt. Die Aushärtung der Zubereitung nach Einbringen in Wasser oder eine wässrige Lösung erfolgt innerhalb weniger Minuten und kann durch gezielte Dosierung von Abbindebeschleuniger, Kristallisationskeimen, Hilfsstoffe und durch die Auswahl der Bestandteile des mineralischen Zementpulvers in weiten Bereichen eingestellt werden. Erfindungsgemäße Zubereitungen mit einem Feststoffgehalt von mehr als 85 Gew.-% zeigen als besonders vorteilhafte Handhabungseigenschaft eine deutlich verringerte Haftung an Handschuhen und Instrumenten und eine sehr gute Modellierbarkeit.

Anhand folgender Ausführungsbeispiele soll die Erfindung näher erläutert werden, ohne die Erfindung auf diese zu beschränken.
Die nachfolgenden Beispiele 1, 2, 4 und 6 wurden mit einem mineralischen Zementpulver (Calciumphosphatzement) der folgenden Zusammensetzung durchgeführt (Angabe in Gew.-% bezogen auf die Masse des mineralischen Zementpulvers):
60 Gew.-% alpha-Tricalciumphosphat, 26 Gew.-% wasserfreies Dicalciumphosphat, 10 Gew.-% Calciumcarbonat und 4 Gew.-% gefälltes Hyroxylapatit.
Im Beispiel 3 wurde ein Magnesium-Calciumphosphatzement (MgCPC) der Struktur Mg_{2,5}Ca_{0,5}(PO4)₂ als mineralisches Zementpulver eingesetzt.
Als organische Trägerflüssigkeit wurde jeweils Miglyol 812, ein gesättigtes teilsynthetisches mittelkettiges Triglycerid, eingesetzt. Folgende Tenside wurden in den Beispielen eingesetzt: anionisches Tensid Amphisol A (Phosphorsäure Monohexadecyl Ester), nichtionisches Tensid Tween80 (Polysorbat 80) und nichtionisches Tensid Cremophor ELP (ethoxyliertes Ricinussöl). Als Abbindebeschleuniger wurden di-Natriumhydrogenphosphat und di-Kaliumhydrogenphosphat eingesetzt. Die Vermahlung erfolgte in Reibschalen oder in Kugelmühlen.

### Beispiel 1: Vergleichsbeispiel Calciumphosphatzement-Zubereitung

20 g CPC-Zementpulver der obengenannten Zusammensetzung wurden mit 4 g Miglyol 812, 300 mg Na₂HPO₄, 500 mg Tween 80 und 200 mg Amphisol A manuell in einer Reibschale vorgemischt. Anschließend wurde die Mischung in einem 100 ml Becher mit 10 Kugeln mit 10 mm Durchmesser (Zirkondioxid-Ausführung) dreimal für 15 min mit jeweils 30 min Pause bei 500 Umdrehungen pro Minute vermischt. Das Ergebnis war eine homogene viskose pastöse, leicht klebrige Zubereitung. Die Zubereitung wurde in eine 10 ml Spritze abgefüllt und anschließend in ein Becherglas mit simulierter Körperflüssigkeit (SBF) (ohne Kanüle) injiziert. Der extrudierte Strang blieb bei leichtem Schütteln im Wesentlichen intakt und härtete in weniger als 60 min soweit aus, dass er ohne Zerfall aus der Flüssigkeit entnommen werden konnte. Nach etwa 24 h war keine Veränderung der Druckfestigkeit mehr feststellbar. Es wurde eine endgültige Druckfestigkeit von 14MPa erreicht.

In einer Laborzentrifuge wurden 5g der vorbeschriebenen Zubereitung in einem Zentrifugenröhrchen bei 1800/min für 15 min zentrifugiert. Nach dieser Zeit bildet sich auf der Oberfläche der Zementpaste ein dünner Ölfilm (geringe Separationsneigung der Zubereitung). Dies ist ein Anzeichen dafür, dass die Zubereitung während der Lagerung dazu neigt, mit zunehmender Lagerung in eine Flüssig- und eine Festphase zu separieren. Die Zubereitung wurde nach der Herstellung in eine 2 ml Einwegspritze (B. Braun Inject® 2ml Luer solo) abgefüllt. Die manuelle Austragung aus dieser Spritze ohne aufgesetzte Kanüle gelang bei mäßigem Kraftaufwand. Es war eine vollständige Austragung aus der Spritze möglich. Die Eigenschaften und Zusammensetzung der Vergleichszubereitung sind in Tabelle 1 ("Vergleich") dargestellt.

### Beispiel 2: Erfindungsgemäße Zubereitung mit Calciumphosphatzement

Es wurden Zubereitungen mit Calciumphosphatzementen (Zubereitungen 1 bis 6 in Tabelle 1) hergestellt, die als organische Trägerflüssigkeit Miglyol 812 enthielten. Es waren zwei Tenside (Cremophor ELP und Amphisol A) enthalten. Als Abbindebeschleuniger wurde di-Kaliumhydrogenphosphat eingesetzt. Die Zubereitungen 1 bis 3 wurden ohne Zusatz von Füllstoffen hergestellt. Die Zubereitungen 4 bis 6 wurden unter Zusatz von Füllstoffen hergestellt (Dicalciumphosphat-Anhydrid in den Zubereitungen 4 und 6, □-Tricalciumphosphat in der Zubereitung 5).

Die Tenside wurden in die Trägerflüssigkeit homogen eingemischt. Anschließend wurden der Calciumphosphatzement und der Abbindebeschleuniger unter Vermahlen (Kugelmühle) beigemischt. Der eingesetzte Calciumphosphatzement bestand aus folgenden Komponenten: 60 Gew.-% α-Tricalciumphosphat, 26 Gew.-% Calciumhydrogenphosphat, 10 Gew.-% Calciumcarbonat, 4 Gew.-% gefälltes Hydroxylapatit.

Dazu wurde das mineralische Zementpulver (Calciumphosphatzement) und der Abbindebeschleuniger in einer Kugelmühle vermischt. Anschließend wurde die flüssige Mischung aus Tensiden und der organischen Trägerflüssigkeit zugegeben und im Mörser mit dem Pulver vermischt, so dass eine homogene viskose Masse erhältlich ist. Die Masse wurde in einer Planetenkugelmühle für insgesamt fünf Stunden vermahlen (im 500 ml-Zirkon-Becher mit 8 Zirkonkugeln einer mittleren Masse von jeweils ca. 110 g). Die Drehzahl wurde dabei schrittweise auf die maximale Drehzahl der Planetenkugelmühle gesteigert.

Für die Zubereitungen 4 bis 6 wurde zu der so erhaltenen Masse der jeweils genannte Füllstoff (Partikelgröße zwischen 63 und 125 µm) zugegeben und durch einfaches Rühren (ohne Vermahlen) eingemischt. Die Zubereitungen lösten sich problemlos von der Wandung des Mischbechers.

In einer Laborzentrifuge wurden jeweils 5 g der verschiedenen Zubereitungen in einem Zentrifugenröhrchen bei 1800/min für 15 min zentrifugiert, um die Separationsneigung der Zubereitungen zu analysieren. In keiner der erfindungsgemäßen Zubereitungen 1 bis 6 bildete sich auf der Oberfläche der Zementpaste ein Ölfilm. Dies ist ein Anzeichen dafür, dass die Zubereitung nicht dazu neigt, mit zunehmender Lagerung in eine Flüssig- und eine Festphase zu separieren.

Die Zubereitungen wurden jeweils in eine 2 ml Einwegspritze abgefüllt. Die manuelle Austragung aus dieser Spritze ohne aufgesetzte Kanüle gelang bei geringem bis mäßigem Kraftaufwand. Es war eine vollständige Austragung aus der Spritze möglich. Die Eigenschaften und Zusammensetzung der unterschiedlichen erfindungsgemäßen Zubereitungen sind in Tabelle 1 dargestellt.

Die Druckfestigkeit der erhaltenen Formkörper wurde an aufrecht stehenden Formkörpern der Dimension 6x6x12 mm mit einer Universalprüfmaschine bei einer Vorschubgeschwindigkeit von 1,0 mm/s bestimmt. Die Formkörper wurden hergestellt, indem die jeweiligen erfindungsgemäßen Zubereitungen in nach oben offene Formen eingebracht wurden und diese anschließend in wässrige 0,9%ige NaCl-Lösung eingelegt wurden. Die Messung der Druckfestigkeit der Formkörper erfolgte nach viertägiger Inkubation in der NaCl-Lösung, wobei zunächst die mit den erfindungsgemäßen Zubereitungen gefüllten Formen für 24h bei 37°C inkubiert wurden und anschließend die entnommenen Formkörper für weitere 72h bei 37°C inkubiert wurden und gleich anschließend gemessen.

**Tabelle 1:**

| | Vergleic h | 1 | 2 | 3 | 4 | 5 | 6 |
|---|---|---|---|---|---|---|---|
| | Gew.-% | Gew.-% | Gew.-% | Gew.-% | Gew.-% | Gew.-% | Gew.-% |
| Zementpulver (CPC) | 80,00 | 83,00 | 82,50 | 83,80 | 62,90 | 62,90 | 54,50 |
| Öl (Miglyol) | 16,00 | 12,30 | 12,20 | 11,10 | 10,00 | 10,00 | 8,67 |
| nichtionisches Tensid* | 2,00 | 2,00 | 2,10 | 2,00 | 1,54 | 1,54 | 1,33 |
| Abbindebeschleuniger** | 1,20 | 2,00 | 2,50 | 2,50 | 1,94 | 1,94 | 1,67 |
| Amphisol A (anionisches Tensid) | 0,80 | 0,70 | 0,70 | 0,60 | 0,62 | 0,62 | 0,54 |
| Füllstoff | 0,00 | 0,00 | 0,00 | 0,00 | 23,00*¹* | 23,00*²* | 33,30*¹* |
| Summe Gew.-% | 100,0 | 100,0 | 100,0 | 100,0 | 100,0 | 100,0 | 100,0 |
| | | | | | | | |
| Gewichtsverhältnis (Feststoff/Flüssigkeit) | 4,32 | 5,67 | 5,67 | 6,30 | 7,22 | 7,22 | 8,49 |
| Feststoffgehalt in Gew.-% | 81,20 | 85,00 | 85,00 | 86,30 | 87,84 | 87,84 | 89,47 |
| Gewichtsanteil Zementpulver und Füllstoff in Gew.-% | 80,00 | 83,00 | 82,50 | 83,80 | 85,90 | 85,90 | 87,80 |
| | | | | | | | |
| Initiale Abbindezeit (min) | 8 | 5 | 4 | 4 | 3 | 3 | 3 |
| Druckfestigkeit nach 100h (MPa) | 14 | 25 | 25 | 28 | 32 | 33 | 39 |
| Ausdrückkraft aus 2,0 ml Spritze | mäßig | gering | gering | gering | mäßig | mäßig | k.a. |
| Separationsneigung | gering | keine | keine | keine | keine | keine | keine |

| | | | | | | | |
|---|---|---|---|---|---|---|---|
| *... Vergleichsbeispiel Tween 80, Zubereitungen 1 bis 6 Cremophor ELP **... Vergleichsbeispiel Na₂HPO₄, Zubereitungen 1 bis 6 K₂HPO₄ *¹*... Dicalciumphosphat-Anhydrid, *²*... □-Tricalciumphosphat k.a.... nicht bestimmt | | | | | | | |

Es wird in Tabelle 1 deutlich, dass die Zubereitungen 1 bis 6 im Vergleich zur Vergleichszubereitung sämtlich eine deutlich verbesserte Druckfestigkeit nach Aushärtung aufweisen und schneller abbinden und zudem die Zubereitungen 1 bis 6 nicht bei Zentrifugation bei hohen Drehzahlen separieren, was eine hohe Lagerungsdauer ermöglicht. Sämtliche Zubereitungen waren aus einer konventionellen Spritze gut und vollständig austragbar. Nach der Aushärtung konnte bei Lagerung in Flüssigkeit keine Separation von Partikeln beobachtet werden.

### Beispiel 3: Erfindungsgemäße Zubereitung mit Magnesiumphosphatzement zur Anwendung in einem Ein-Pasten-System oder Zwei-Komponenten-System mit wasserfreier und wasserenthaltender Paste

Es wurden Zubereitungen mit Magnesium-Calciumphosphatzement (Zubereitungen 7 bis 11 in Tabelle 2) hergestellt. Als mineralisches Zementpulver wurde Magnesium-Calciumphosphatzement (MgCPC) der Struktur Mg_{2,5}Ca_{0,5}(PO4)₂ eingesetzt. Dieses wurde aus CaCO₃ und MgCO₃ im molaren Verhältnis
(Ca+Mg): (PO₄) = 3 : 2
durch Brennen bei 1050 °C und anschließende Vermahlung in einer Planetenkugelmühle in Bechern und mittels Mahlkugeln aus Zirkondioxid hergestellt.

Für die Herstellung einer pastösen Zubereitung wurde als organische Trägerflüssigkeit Miglyol 812, ein gesättigtes teilsynthetisches mittelkettiges Triglycerid, eingesetzt. Folgende Tenside wurden eingesetzt: anionisches Tensid Amphisol A (Phosphorsäure Monohexadecyl Ester), nichtionisches Tensid Tween80 (Polysorbat 80) und nichtionisches Tensid Cremophor ELP (ethoxyliertes Ricinussöl). Die Tenside wurden in die Trägerflüssigkeit homogen eingemischt.

Das MgCPC-Pulver wurde mit (NH₄)₂HPO₄ und (NH₄)H₂PO₄ in einer Planetenkugelmühle mit Zirkonausstattung mit der tensidhaltigen organischen Trägerflüssigkeit vermischt und vermahlen. Für die Herstellung der Zubereitungen 8 und 11 wurde im Anschluss daran zu der erhaltenen Paste CaHPO₄ einer durchschnittlichen Partikelgröße von 85 µm (Siebfraktion) zugemischt.

Zur Auslösung der Aushärtereaktion wurden die erhaltenen pastösen Zubereitungen im jeweils in Tabelle 2 angegebenen Verhältnis mit Wasser (Ein-Pasten-System: Zubereitungen 7, 9 und 10) oder einer wässrigen Paste enthaltend 35 Gew.-% nanokristallines Hydroxylapatit (Zwei-Komponenten-System: Zubereitungen 8 und 11) vermischt. Die Zubereitungen 9 bis 11 wurden dazu in die größere Kammer einer Doppelkammerkartusche eingefüllt, wobei die jeweilige wässrige Zubereitung in die kleinere Kammer eingefüllt wurde. Die Inhalte beider Kartuschen wurden dann parallel durch einen Zwangsmischer gedrückt und dabei homogen vermischt. Abbindezeit, Druckfestigkeit, Separationsneigung und Ausdrückkraft wurden wie in Beispiel 1 ermittelt. Die Zusammensetzungen und Ergebnisse sind in Tabelle 2 dargestellt.

**Tabelle 2:**

| | 7 | 8 | 9 | 10 | 11 |
|---|---|---|---|---|---|
| | Gew.-% | Gew.-% | Gew.-% | Gew.-% | Gew.-% |
| **Zementzubereitung** | | | | | |
| Zementpulver (MgCPC) | 75,11 | 58,39 | 75,11 | 75,11 | 58,39 |
| Öl (Miglyol) | 10,0 | 11,27 | 10,0 | 10,0 | 11,27 |
| CremophorELP (nichtionisches Tensid) | 1,58 | 1,78 | 1,58 | 1,58 | 1,78 |
| (NH₄)₂HPO₄ | 7,49 | 6,08 | 7,49 | 7,49 | 6,08 |
| (NH₄)H₂PO₄ | 4,98 | 4,04 | 4,98 | 4,98 | 4,04 |
| Amphisol A (anionisches Tensid) | 0,84 | 0,92 | 0,84 | 0,84 | 0,92 |
| Füllstoff CaHPO₄ (Ø 85 µm) | 0,00 | 17,52 | 0,00 | 0,00 | 17,52 |
| Summe Gew.-% | 100,00 | 100,00 | 100,00 | 100,00 | 100,00 |
| | | | | | |
| Gewichtsverhältnis (Feststoff/Flüssigkeit) | 7,05 | 6,16 | 7,05 | 7,05 | 6,16 |
| | | | | | |
| Gewichtsanteil Zementpulver und Füllstoff in Gew.-% | 87,58 | 86,03 | 87,58 | 87,58 | 86,03 |
| | | | | | |

| **Wässrige Zubereitung** | | | | | |
|---|---|---|---|---|---|
| Wasser | 100 | 65 | 100 | 100 | 65 |
| Füllstoff nanokristallines Hydroxylapatit | 0 | 35 | 0 | 0 | 35 |
| | | | | | |
| Mischungsverhältnis Zementzubereitung: wässrige Zubereitung (Volumenverhältnis) | 10:1 | 10:1 | 10:1 | 4:1 | 4:1 |
| Vermischung | Rühren | Rühren | DKS | DKS | DKS |
| | | | | | |
| Initiale Abbindezeit (min) | 6 | 4 | 4 | 6 | 4 |
| Druckfestigkeit (MPa) | 56*³* | 52*¹* | 48*²* | 42*²* | 57*²* |
| Ausdrückkraft aus 2,0 ml Spritze (N) | k.a. | k.a. | 85 | 55 | 83 |
| Separationsneigung | keine | keine | keine | keine | keine |

| | | | | | |
|---|---|---|---|---|---|
| DKS... Doppelkammerspritze mit Zwangsmischer; *¹*... bestimmt nach 100h, *²*.... bestimmt nach 6h, *³*... bestimmt nach 18h; k.a.... nicht bestimmt | | | | | |

Sämtliche Zubereitungen waren aus einer konventionellen Spritze gut und vollständig austragbar. Nach der Aushärtung konnte bei Lagerung in Flüssigkeit keine Separation von Partikeln beobachtet werden.

### Beispiel 4: Erfindungsgemäße Zubereitung mit Calciumphosphatzement zur Anwendung in einem Zwei-Komponenten-System mit wasserfreier und Wasser enthaltender Paste

Es wurden Zubereitungen mit Calciumphosphatzementen (Zubereitungen 12 bis 14 in Tabelle 3) hergestellt, die als organische Trägerflüssigkeit Miglyol 812 (ein halbsynthetisches Öl) enthalten. Es sind zwei Tenside (Cremophor ELP und Amphisol A) enthalten. Als Abbindebeschleuniger wurde di-Kaliumhydrogenphosphat eingesetzt. Die Tenside wurden in die Trägerflüssigkeit homogen eingemischt. Anschließend wurden der Calciumphosphatzement und der Abbindebeschleuniger unter Vermahlen (Kugelmühle) beigemischt. Der eingesetzte Calciumphosphatzement bestand aus 60 Gew.-% α-Tricalciumphosphat, 26 Gew.-% Calciumhydrogenphosphat, 10 Gew.-% Calciumcarbonat, 4 Gew.-% gefällte, Hydroxylapatit. Zudem wurde eine Vergleichszubereitung mit Calciumphosphatzement derselben Zusammensetzung hergestellt, deren Rezeptur in Tabelle 3 angegeben ist.

Zur Auslösung der Aushärtereaktion wurden die erhaltenen pastösen Zubereitungen im Gewichtsverhältnis von 4 : 1 mit einer pastösen wässrigen Lösung von 6 Gew.-% Hydroxyethylstärke (wässrige Zubereitung) vermischt. Dazu wurden die Zubereitungen (Vergleichszubereitung, erfindungsgemäße Zubereitungen 12 bis 14) in die größere Kammer einer Doppelkammerkartusche und die wässrige Zubereitung in die kleinere Kammer eingefüllt. Die Inhalte beider Kartuschen wurden dann parallel durch einen Zwangsmischer gedrückt und dabei homogen vermischt. Abbindezeit, Druckfestigkeit, Separationsneigung und Ausdrückkraft wurden wie in Beispiel 1 ermittelt. Die Zusammensetzungen und Ergebnisse sind in Tabelle 3 dargestellt.

**Tabelle 3:**

| | Vergleich | 12 | 13 | 14 |
|---|---|---|---|---|
| | Gew.-% | Gew.-% | Gew.-% | Gew.-% |
| **Zementzubereitung** | | | | |
| Zementpulver (CPC) | 79,00 | 83,00 | 84,00 | 84,00 |
| Öl (Miglyol) | 17,00 | 13,50 | 12,70 | 12,00 |
| nichtionisches Tensid* | 2,00 | 2,00 | 2,10 | 2,00 |
| Abbindebeschleuniger** | 1,00 | 0,70 | 0,50 | 1,30 |
| Amphisol A (anionisches Tensid) | 1,00 | 0,80 | 0,70 | 0,70 |
| Summe Gew.-% | 100,00 | 100,00 | 100,00 | 100,00 |
| | | | | |
| Gewichtsverhältnis (Feststoff/Flüssigkeit) | 4,00 | 5,13 | 5,45 | 5,80 |
| Feststoffgehalt in Gew.-% | 80,00 | 83,70 | 84,50 | 85,30 |
| Gewichtsanteil Zementpulver (= Feststoffgehalt ohne Abbindebeschleuniger) in Gew.-% | 79,00 | 83,00 | 84,00 | 84,00 |
| | | | | |

| **Wässrige Zubereitung** | | | | |
|---|---|---|---|---|
| Wasser | 94 | 94 | 94 | 94 |
| Füllstoff Hydroxyethylstärke | 6 | 6 | 6 | 6 |
| | | | | |
| Mischungsverhältnis Zementzubereitung: wässrige Zubereitung (Volumenverhältnis) | 4:1 | 4:1 | 4:1 | 4:1 |
| Vermischung | DKS | DKS | DKS | DKS |
| | | | | |
| Initiale Abbindezeit (min) | 4,5 | 3,5 | 4,0 | 3,0 |
| Druckfestigkeit nach 100h (MPa) | 15,4 | 21,3 | 23,8 | 24,6 |
| Ausdrückkraft aus 2,0 ml Spritze (N) | 85 | 55 | 58 | 67 |
| Separationsneigung | gering | keine | keine | keine |

| | | | | |
|---|---|---|---|---|
| *... Vergleichsbeispiel Tween 80, Zubereitungen 12 bis 14 CremophorELP **... Vergleichsbeispiel Na₂HPO₄, Zubereitungen 12 bis 14 K₂HPO₄ | | | | |

Die Zubereitungen 12 bis 14 wiesen nach Aushärtung im Vergleich zur bekannten Zementzubereitung ("Vergleich") eine verbesserte Druckfestigkeit auf. Zudem konnte eine schnelle initiale Abbindung beobachtet werden. Die Zubereitungen 12 bis 14 separierten nicht bei Zentrifugation bei hohen Drehzahlen. Sämtliche Zubereitungen waren aus einer konventionellen Spritze gut und vollständig austragbar. Nach der Aushärtung konnte bei Lagerung in Flüssigkeit keine Separation von Partikeln beobachtet werden.

### Beispiel 5: Erfindungsgemäße Zubereitung mit strontriumhaltigen Calciumphosphatzement

Es wurde ein calciumionenhaltiges mineralisches Zementpulver (strontiumhaltiger Calciumphosphatzement) der folgenden Zusammensetzung hergestellt (Angabe in Gew.-% bezogen auf die Masse des mineralischen Zementpulvers):
60 Gew.-% alpha-Tricalciumphosphat, 26 Gew.-% wasserfreies Dicalciumphosphat, 10 Gew.-% Strontiumcarbonat und 4 Gew.-% gefälltes Hyroxylapatit.

Es wurde analog zu Beispiel 2 in einer Planetenkugelmühle eine Zubereitung folgender Zusammensetzung hergestellt: 82,5 Gew.-% mineralisches Zementpulver, 12,2 Gew.-% Miglyol 812, 2,1 Gew.-% Cremophor ELP, 2,5 Gew.-% K₂HPO₄, 0,7 Gew.-% Amphisol A (dies entspricht einem Feststoff/Flüssigkeits-Gewichtsverhältnis von 5,67, einem Feststoffanteil von 85 Gew.-% und einem Gewichtsanteil von Zementpulver und Füllstoff in der Zubereitung von 82,5 Gew.-%). Abbindezeit, Druckfestigkeit und Separationsneigung der so erhaltenen Zubereitung wurden wie in Beispiel 1 ermittelt. Die initiale Abbindezeit betrug 5 min. Die Druckfestigkeit nach 100 h betrug 38 MPa. Es wurde keine Separationsneigung und Partikelfreisetzung beobachtet.

### Beispiel 6: Erfindungsgemäße Zubereitung mit Calciumphosphatzement und Zusatz mineralischer Glasfasern

Eine Calciumphosphatzement-Paste (CPC-Paste) mit der Pulverzusammensetzung und der Zusammensetzung der Trägerflüssigkeit wie in Beispiel 2 wurde auf ein Pulver: Trägerflüssigkeits-Verhältnis von 85:15 eingestellt. In 95g dieser CPC-Paste wurden 5g einer Kieselsäurefaser mit 7,5µm Durchmesser und ca. 3mm Länge eingearbeitet. Die CPC-Paste war anschließend noch gut mit dem Spatel verarbeitbar. Der Feststoffgehalt der Glasfaserenthaltenden Paste betrug 85,75%. Die Präparation der Probekörper für die Bestimmung der Druckfestigkeit sowie die Bestimmung der Druckfestigkeit erfolgte wie in Ausführungsbeispiel 2 beschrieben.

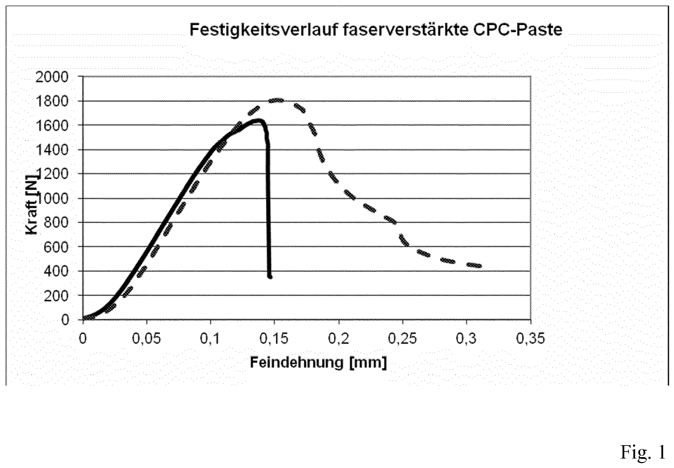

Fig. 1 zeigt den Druckfestigkeitsverlauf über die Deformation (Kraft-Dehnungs-Kurve). Es ist deutlich zu sehen, dass sich das Deformationsverhalten der faserverstärkten Probe (gestrichelte Kurve) deutlich von der unverstärkten Probe (durchgezogene Kurve) unterscheidet. Während die unverstärkte Probe eine hohe Druckfestigkeit erreicht, fällt diese nach Überschreiten der maximalen Druckkraft katastrophal ab; der Formkörper wird dabei völlig zerstört. Die verstärkte Probe zeigt ein deutlich anderes Verhalten. Die maximale Drucklast ist weiter erhöht. Nach deren Überschreiten erfolgt ein wesentlich langsamerer Abfall und der Formkörper kann auch bei weiterer Verformung noch erheblich Kräfte aufnehmen. Der Formkörper wird zwar deformiert, bleibt aber über einen weiten Verformungsbereich als solcher zusammenhängend.

## Patentansprüche

1. Zubereitung zur Herstellung eines Implantats, vorzugsweise Knochenimplantats, umfassend:
a) mineralisches Zementpulver, das mindestens eine Calciumionen-haltige und/oder mindestens eine Magnesiumionen-haltige anorganische Verbindung als Reaktivkomponente enthält,
b) mindestens eine organische Trägerflüssigkeit, wobei die organische Trägerflüssigkeit eine schwer wasserlösliche Trägerflüssigkeit ist, wobei die schwer wasserlösliche Trägerflüssigkeit ausgewählt ist aus kurz- oder mittelkettigen Triglyceriden,
wobei kurzkettige Triglyceride Verbindungen von Fettsäuren einer Länge von jeweils 2 bis 5 Kohlenstoffatomen sind,
wobei mittelkettige Triglyceride Verbindungen von Fettsäuren einer Länge von jeweils 6 bis 14 Kohlenstoffatomen sind,
c) mindestens zwei Tenside ausgewählt aus mindestens zwei der Gruppen der anionischen, kationischen, amphoteren und nichtionischen Tenside,
d) weniger als 1 Gew.-% Wasser bezogen auf die Gesamtmasse der Zubereitung,
**dadurch gekennzeichnet,**
**dass** das Gewichtsverhältnis der insgesamt in der Zubereitung enthaltenen Feststoffe zur Summe des Gewichts der organischen Trägerflüssigkeit und der mindestens zwei Tenside mehr als 5 beträgt, wenn das Zementpulver Calciumionen-haltige Verbindungen und keine Magnesiumionen-haltigen Verbindungen als Reaktivkomponente enthält, oder
mehr als 6 beträgt, wenn das Zementpulver Magnesiumionen-haltige oder Magnesiumionen- und Calciumionen-haltige Verbindungen als Reaktivkomponenten enthält, und
**dass** mindestens ein anionisches und mindestens ein nichtionisches Tensid enthalten ist, wobei das mindestens eine anionische und mindestens eine nichtionische Tensid mindestens ein ethoxylierter Fettalkohol, mindestens eine ethoxylierte Fettsäure, mindestens ein ethoxylierter Sorbitanfettsäureester, mindestens ein ethoxyliertes Fett oder mindestens ein ethoxyliertes Öl und
mindestens ein mit Zitronensäure, Schwefelsäure oder Phosphorsäure veresterter Fettalkohol, mindestens ein mit Zitronensäure, Schwefelsäure oder Phosphorsäure verestertes Mono- oder Diglycerid oder Salz davon oder mindestens eine mit Zitronensäure, Schwefelsäure oder Phosphorsäure veresterte Fettsäure oder Salze davon sind.

2. Zubereitung nach Anspruch 1, **dadurch gekennzeichnet, dass** nach Aushärtung der Zubereitung in einem wässrigen Medium erhältliche Formkörper eine Druckfestigkeit von mehr als 25 MPa aufweisen,
wobei die Formkörper durch Einbringen der Zubereitungen in nach oben offenen Formen und Einlegen in eine wässrige 0,9%ige NaCl-Lösung hergestellt werden,
wobei die Messung der Druckfestigkeit der Formkörper nach viertägiger Inkubation in der NaCl-Lösung erfolgt,
wobei zunächst die mit den Zubereitungen gefüllten Formen für 24 h bei 37°C inkubiert und anschließend die entnommenen Formkörper für weitere 72 h bei 37°C inkubiert werden,
wobei die Messung unmittelbar nach der Inkubation im noch feuchten Zustand erfolgt,
wobei die Druckfestigkeit der stehenden Formkörper der Dimension 6x6x12 mm entlang deren längster Achse mit einer Universalprüfmaschine bei einer Vorschubgeschwindigkeit von 1,0 mm/s bestimmt wird.

3. Zubereitung nach Anspruch 1 oder 2, wobei die Zubereitung einen Gesamtfeststoffgehalt von 80 bis 95 Gew.-% aufweist.

4. Zubereitung nach einem der vorhergehenden Ansprüche, wobei mindestens ein Abbindebeschleuniger enthalten ist, vorzugsweise ausgewählt aus Phosphatsalzen, organischen Säuren oder Salzen organischer Säuren, bevorzugt kaliumionenhaltigen Phosphaten, wobei die Masse des Abbindebeschleunigers 0,1 bis 5 % der Masse des mineralischen Zementpulvers entspricht.

5. Zubereitung nach einem der Ansprüche 1 bis 3, **dadurch gekennzeichnet, dass** bei Verwendung von Magnesiumionen- oder Magnesiumionen- und Calciumionen- haltigen Verbindungen als Reaktivkomponente die Zubereitung zusätzlich Hydrogenphosphate enthält.

6. Zubereitung nach einem der vorhergehenden Ansprüche, wobei mindestens ein hydrophiles Tensid mit einem HLB-Wert von mehr als 8 und mindestens ein lipophiles Tensid mit einem HLB-Wert von weniger als 5 enthalten ist.

7. Zubereitung nach einem der vorhergehenden Ansprüche, wobei mindestens ein Füllstoff enthalten ist, ausgewählt aus Strontiumcarbonat, Strontiumhydrogenphosphat, Strontiumphosphat, Glaskeramiken, Calciumcarbonat, Carboxymethylstärke, Eisenoxiden, Siliciumdioxiden, Bariumsulfat, Glycerinstearat, gefälltem nanokristallinen Hydroxylapatit, calciumdefizienten Hydroxylapatit und Tricalciumphosphat und mineralischen oder organischen Fasern.

8. Zubereitung nach einem der vorhergehenden Ansprüche, wobei die Gesamtmasse der nichtionischen Tenside vorzugsweise mindestens das Doppelte der Gesamtmasse der anionischen Tenside beträgt.

9. Zubereitung nach einem der vorhergehenden Ansprüche zur Verwendung zur Herstellung als Material zur Behandlung von Knochendefekten oder zum Knochenaufbau, zur Befestigung von Knochenimplantaten oder zur Herstellung von implantierbaren Wirkstoffträgern.

10. Fester Formkörper, erhältlich durch Einbringen einer Zubereitung nach einem der Ansprüche 1 bis 8 in eine Wasser enthaltende Zubereitung oder durch Vermischen einer Zubereitung nach einem der Ansprüche 1 bis 8 mit einer Wasser enthaltenden Zubereitung.

11. Verfahren zur Herstellung einer Zubereitung nach einem der Ansprüche 1 bis 8, wobei mineralisches Zementpulver oder einzelne Bestandteile des mineralischen Zementpulvers sukzessive durch Vermahlung in der organischen Trägerflüssigkeit in Gegenwart der mindestens zwei Tenside dispergiert werden, wobei die organische Trägerflüssigkeit eine schwer wasserlösliche Trägerflüssigkeit ist, wobei die schwer wasserlösliche Trägerflüssigkeit ausgewählt ist aus kurz- oder mittelkettigen Triglyceriden,
wobei kurzkettige Triglyceride Verbindungen von Fettsäuren einer Länge von jeweils 2 bis 5 Kohlenstoffatomen sind,
wobei mittelkettige Triglyceride Verbindungen von Fettsäuren einer Länge von jeweils 6 bis 14 Kohlenstoffatomen sind,
**dadurch gekennzeichnet,**
**dass** das Gewichtsverhältnis der insgesamt in der Zubereitung enthaltenen Feststoffe zur Summe des Gewichts der organischen Trägerflüssigkeit und der mindestens zwei Tenside mehr als 5 beträgt, wenn das Zementpulver Calciumionen-haltige Verbindungen und keine Magnesiumionen-haltigen Verbindungen als Reaktivkomponente enthält, oder
mehr als 6 beträgt, wenn das Zementpulver Magnesiumionen-haltige oder Magnesiumionen- und Calciumionen-haltige Verbindungen als Reaktivkomponenten enthält, und
**dass** mindestens ein anionisches und mindestens ein nichtionisches Tensid enthalten ist,
wobei das mindestens eine anionische und mindestens eine nichtionische Tensid mindestens ein ethoxylierter Fettalkohol, mindestens eine ethoxylierte Fettsäure, mindestens ein ethoxylierter Sorbitanfettsäureester, mindestens ein ethoxyliertes Fett oder mindestens ein ethoxyliertes Öl und
mindestens ein mit Zitronensäure, Schwefelsäure oder Phosphorsäure veresterter Fettalkohol, mindestens ein mit Zitronensäure, Schwefelsäure oder Phosphorsäure verestertes Mono- oder Diglycerid oder Salz davon oder mindestens eine mit Zitronensäure, Schwefelsäure oder Phosphorsäure veresterte Fettsäure oder Salze davon sind.

12. Verfahren nach Anspruch 11, **dadurch gekennzeichnet, dass** weitere Bestandteile des mineralischen Zementpulvers und/oder Füllstoffe, jeweils mit einer Partikelgröße von weniger als 10 µm, unter ständigem Vermahlen zugegeben werden.

13. Verfahren nach Anspruch 12, **dadurch gekennzeichnet, dass** anschließend grobe Füllstoffe mit einer Partikelgröße von mehr als 50 µm zugegeben und vermischt werden.

## Claims

1. Preparation for producing an implant, preferably a bone implant, comprising:
a) mineral cement powder which contains at least one calcium ion-containing and/or at least one magnesium ion-containing inorganic compound as a reactive component,
b) at least one organic carrier liquid, the organic carrier liquid being a carrier liquid of low solubility in water, the carrier liquid of low solubility in water being selected from short-chain or medium-chain triglycerides, short-chain triglycerides being compounds of fatty acids each having a length of from 2 to 5 carbon atoms, medium-chain triglycerides being compounds of fatty acids each having a length of from 6 to 14 carbon atoms,
c) at least two surfactants selected from at least two of the groups of anionic, cationic, amphoteric and non-ionic surfactants,
d) less than 1 wt.% water based on the total weight of the preparation,
**characterised in that** the weight ratio of the total solids contained in the preparation to the sum of the weight of the organic carrier liquid and the at least two surfactants is greater than 5 if the cement powder contains calcium ion-containing compounds and does not contain magnesium ion-containing compounds as a reactive component, or is greater than 6 if the cement powder contains magnesium-ion containing compounds or magnesium ion-containing and calcium ion-containing compounds as reactive components, and **in that** at least one anionic and at least one non-ionic surfactant are contained, the at least one anionic and at least one non-ionic surfactant being at least one ethoxylated fatty alcohol, at least one ethoxylated fatty acid, at least one ethoxylated sorbitan fatty acid ester, at least one ethoxylated fat or at least one ethoxylated oil and at least one fatty alcohol esterified with citric acid, sulphuric acid or phosphoric acid, at least one mono or diglyceride esterified with citric acid, sulphuric acid or phosphoric acid, or a salt thereof, or at least one fatty acid esterified with citric acid, sulphuric acid or phosphoric acid, or salts thereof.

2. Preparation according to claim 1, **characterised in that** moulded bodies, which can be obtained after hardening the preparation in an aqueous medium, have a compressive strength greater than 25 MPa, the moulded bodies being produced by introducing the preparations into moulds that are open at the top and placing into an aqueous 0.9% NaCl solution, the compressive strength of the moulded bodies being measured after a four-day incubation in the NaCl solution, the moulds which have been filled with the preparations first being incubated for 24 hours at 37°C and the obtained moulded bodies subsequently being incubated for a further 72 hours at 37°C, the measurement being taken immediately after incubation in a still wet state, the compressive strength of the upright moulded bodies, which have dimensions of 6 x 6 x 12 mm, being determined along the longest axis thereof by using a universal testing machine at a feed rate of 1.0 mm/s.

3. Preparation according to either claim 1 or claim 2, wherein the preparation has a total solids content of from 80 to 95 wt.%.

4. Preparation according to any of the preceding claims, wherein at least one accelerating admixture is contained, preferably selected from phosphate salts, organic acids or salts of organic acids, preferably potassium ion-containing phosphates, wherein the weight of the accelerating admixture corresponds to from 0.1 to 5% of the weight of the mineral cement powder.

5. Preparation according to any of claims 1 to 3, **characterised in that** the preparation additionally contains hydrogen phosphates when using magnesium ion-containing compounds or magnesium ion-containing and calcium ion-containing compounds as a reactive component.

6. Preparation according to any of the preceding claims, wherein at least one hydrophilic surfactant having an HLB value greater than 8 and at least one lipophilic surfactant having an HLB value of less than 5 are contained.

7. Preparation according to any of the preceding claims, wherein at least one filler is contained, selected from strontium carbonate, strontium hydrogen phosphate, strontium phosphate, glass ceramics, calcium carbonate, carboxymethyl starch, iron oxides, silicon dioxides, barium sulphate, glyceryl stearate, precipitated nanocrystalline hydroxylapatite, calcium-deficient hydroxylapatite and tricalcium phosphate and mineral or organic fibres.

8. Preparation according to any of the preceding claims, wherein the total weight of the non-ionic surfactants is preferably at least double the total weight of the anionic surfactants.

9. Preparation according to any of the preceding claims for use in production as a material for treating bone defects or for forming bone, for attaching bone implants or for producing active substance carriers which can be implanted.

10. Solid moulded body, which can be obtained by introducing a preparation according to any of claims 1 to 8 into a preparation containing water or by mixing a preparation according to any of claims 1 to 8 with a preparation containing water.

11. Method for producing a preparation according to any of claims 1 to 8, mineral cement powder or individual constituents of the mineral cement powder being gradually dispersed by grinding in the organic carrier liquid in the presence of the at least two surfactants, the organic carrier liquid being a carrier liquid of low solubility in water, the carrier liquid of low solubility in water being selected from short-chain or medium-chain triglycerides, short-chain triglycerides being compounds of fatty acids each having a length of from 2 to 5 carbon atoms, medium-chain triglycerides being compounds of fatty acids each having a length of from 6 to 14 carbon atoms, **characterised in that** the weight ratio of the total solids contained in the preparation to the sum of the weight of the organic carrier liquid and the at least two surfactants is greater than 5 if the cement powder contains calcium ion-containing compounds and does not contain magnesium ion-containing compounds as a reactive component, or is greater than 6 if the cement powder contains magnesium ion-containing compounds or magnesium ion-containing and calcium ion-containing compounds as reactive components, and **in that** at least one anionic and at least one non-ionic surfactant are contained, the at least one anionic and at least one non-ionic surfactant being at least one ethoxylated fatty alcohol, at least one ethoxylated fatty acid, at least one ethoxylated sorbitan fatty acid ester, at least one ethoxylated fat or at least one ethoxylated oil and at least one fatty alcohol esterified with citric acid, sulphuric acid or phosphoric acid, at least one mono or diglyceride esterified with citric acid, sulphuric acid or phosphoric acid, or a salt thereof, or at least one fatty acid esterified with citric acid, sulphuric acid or phosphoric acid, or salts thereof.

12. Method according to claim 11, **characterised in that** further constituents of the mineral cement powder and/or fillers, each having a particle size of less than 10 µm, are added with continuous grinding.

13. Method according to claim 12, **characterised in that** coarse fillers having a particle size of greater than 50 µm are subsequently added and mixed.

## Revendications

1. Préparation, destinée à la fabrication d'un implant, de préférence d'un implant osseux, comprenant:
a) une poudre de ciment minéral qui contient comme composant réactif au moins un composé minéral contenant des ions calcium et/ou au moins un composé minéral contenant des ions magnésium,
b) au moins un liquide porteur organique, le liquide porteur organique étant un liquide porteur peu soluble dans l'eau, le liquide porteur peu soluble dans l'eau étant choisi parmi les triglycérides à chaîne courte ou à chaîne moyenne,
les triglycérides à chaîne courte étant des composés d'acides gras qui ont chacun une longueur de 2 à 5 atomes de carbone,
les triglycérides à chaîne moyenne étant des composés d'acides gras qui ont chacun une longueur de 6 à 14 atomes de carbone,
c) au moins deux tensioactifs choisis parmi au moins deux des groupes de tensioactifs anioniques, cationiques, amphotères et non ioniques,
d) moins de 1 % en poids d'eau, par rapport à la masse totale de la préparation,
**caractérisée en ce que**
le rapport en poids de toutes les matières solides contenues dans la préparation à la somme du poids du liquide porteur organique et des au moins deux tensioactifs est supérieur à 5 lorsque la poudre de ciment contient des composés contenant des ions calcium et ne contient aucun composé contenant des ions magnésium comme composant réactif, ou est supérieur à 6 lorsque la poudre de ciment contient des composés contenant des ions magnésium ou des ions magnésium et des ions calcium comme composants réactifs, et **en ce que** au moins un tensioactif anionique et au moins un tensioactif non ionique sont contenus,
l'au moins un tensioactif anionique et l'au moins un tensioactif non ionique étant au moins un alcool gras éthoxylé, au moins un acide gras éthoxylé, au moins un ester d'acides gras de sorbitan éthoxylé, au moins un corps gras éthoxylé ou au moins une huile éthoxylée et
au moins un alcool gras estérifié avec de l'acide citrique, de l'acide sulfurique ou de l'acide phosphorique, au moins un monoglycéride ou diglycéride ou un sel de ceux-ci estérifié avec de l'acide citrique, de l'acide sulfurique ou de l'acide phosphorique ou au moins un acide gras ou des sels de celui-ci estérifiés avec de l'acide citrique, de l'acide sulfurique ou de l'acide phosphorique.

2. Préparation selon la revendication 1, **caractérisée en ce que** des corps moulés, pouvant être obtenus après durcissement de la préparation dans un milieu aqueux, présentent une résistance à la compression supérieure à 25 MPa,
les corps moulés étant fabriqués par introduction des préparations dans des moules ouverts vers le haut et par placement de ceux-ci dans une solution aqueuse à 0,9 % de NaCl,
la mesure de la résistance à la compression des corps moulés étant effectuée après quatre jours d'incubation dans la solution de NaCl,
les moules remplis avec les préparations étant d'abord incubés pendant 24 h à 37 °C puis les corps moulés retirés étant incubés pendant encore 72 heures à 37 °C,
la mesure étant effectuée immédiatement après l'incubation à l'état encore humide,
la résistance à la compression des corps moulés verticaux de dimensions 6x6x12 mm étant déterminée le long de leur grand axe avec une machine d'essai universelle à une vitesse de progression de 1,0 mm/s.

3. Préparation selon la revendication 1 ou 2, la préparation ayant une teneur totale en matières solides de 80 à 95 % en poids.

4. Préparation selon l'une des revendications précédentes, contenant au moins un accélérateur de durcissement, de préférence choisi parmi les sels de phosphate, les acides organiques ou les sels d'acides organiques, de préférence les phosphates contenant des ions calcium, la masse de l'accélérateur de prise correspondant à un pourcentage allant de 0,1 à 5 % de la masse de la poudre de ciment minéral.

5. Préparation selon l'une des revendications 1 à 3, **caractérisée en ce que**, lors de l'utilisation de composés contenant des ions magnésium ou des ions magnésium et des ions calcium comme composants réactifs, la préparation contient en plus des hydrogénophosphates.

6. Préparation selon l'une des revendications précédentes, contenant au moins une tensioactif hydrophile ayant une valeur HLB supérieure à 8 et au moins un tensioactif lipophile ayant une valeur HLB inférieure à 5.

7. Préparation selon l'une des revendications précédentes, contenant au moins une charge choisie parmi le carbonate de strontium, l'hydrogénophosphate de strontium, le phosphate de strontium, les vitrocéramiques, le carbonate de calcium, le carboxyméthylamidon, les oxydes de fer, les silices, le sulfate de baryum, le stéarate de glycérol, l'hydroxyapatite nanocristalline précipitée, l'hydroxyapatite à déficience en calcium et le phosphate tricalcique et des fibres minérales ou organiques.

8. Préparation selon l'une des revendications précédentes, la masse totale des tensioactifs non ioniques étant égale de préférence à au moins deux fois la masse totale des tensioactifs anioniques.

9. Préparation selon l'une des revendications précédentes destiné à être utilisée dans la production de matière pour le traitement de défauts osseux ou pour la formation osseuse, pour la fixation d'implants osseux ou pour la production de supports de principes actifs implantables.

10. Corps moulé solide, pouvant être obtenu par introduction d'une préparation selon l'une des revendications 1 à 8 dans une préparation contenant de l'eau ou par mélange d'une préparation selon l'une des revendications 1 à 8 avec une préparation contenant de l'eau.

11. Procédé de production d'une préparation selon l'une des revendications 1 à 8, de la poudre de ciment minéral ou des constituants individuels de la poudre de ciment minéral étant dispersés successivement par broyage dans le liquide porteur organique en présence d'au moins deux tensioactifs, le liquide porteur organique étant un liquide porteur peu soluble dans l'eau, le liquide porteur peu soluble dans l'eau étant choisi parmi les triglycérides à chaîne courte ou à chaîne moyenne,
les triglycérides à chaîne courte étant des composés d'acides gras qui ont chacun une longueur de 2 à 5 atomes de carbone,
les triglycérides à chaîne moyenne étant des composés d'acides gras qui ont chacun une longueur de 6 à 14 atomes de carbone,
**caractérisé en ce que**
le rapport en poids de toutes les matières solides contenues dans la préparation à la somme du poids du liquide porteur organique et des au moins deux tensioactifs est supérieur à 5 lorsque la poudre de ciment contient des composés contenant des ions calcium et ne contient aucun composé contenant des ions magnésium comme composant réactif, ou est supérieur à 6 lorsque la poudre de ciment contient des composés contenant des ions magnésium ou des ions magnésium et des ions calcium comme composants réactifs, et **en ce que**
au moins un tensioactif anionique et au moins un tensioactif non ionique sont contenus,
l'au moins un tensioactif anionique et l'au moins un tensioactif non ionique étant au moins un alcool gras éthoxylé, au moins un acide gras éthoxylé, au moins un ester d'acides gras de sorbitan éthoxylé, au moins un corps gras éthoxylé ou au moins une huile éthoxylée et au moins un alcool gras estérifié avec de l'acide citrique, de l'acide sulfurique ou de l'acide phosphorique, au moins un monoglycéride ou diglycéride ou un sel de ceux-ci estérifié avec de l'acide citrique, de l'acide sulfurique ou de l'acide phosphorique ou au moins un acide gras ou des sels de celui-ci estérifiés avec de l'acide citrique, de l'acide sulfurique ou de l'acide phosphorique.

12. Procédé selon la revendication 11, **caractérisé en ce que** d'autres constituants de la poudre de ciment minéral et/ou des charges, ayant chacun une granulométrie inférieure à 10 µm, sont ajoutés avec un broyage constant.

13. Procédé selon la revendication 12, **caractérisé en ce que** des charges grossières ayant une granulométrie supérieure à 50 µm sont ajoutées et mélangées.
